# EUROPEAN PATENT APPLICATION

(11) **EP 3 744 177 A1**
(43) Date of publication of application: **02.12.2020**
(21) Application number: 20167323.3
(22) Date of filing: 24.04.2015
(51) Int. Cl.: A01N 63/00, C12N 15/86

(54) **AAV-BASED GENE THERAPY FOR MULTIPLE SCLEROSIS**

(30) Priority: 24.04.2014 US 201461983924 P
(62) Divisional of application: 15782332.9
(71) Applicant: University of Florida Research Foundation, Inc., Gainesville, FL 32611 (US)
(72) Inventor: HOFFMAN, Brad E., Gainesville, FL 32606 (US)
(74) Representative: Potter Clarkson

(57) **Abstract**

Disclosed are AAV viral-based vector compositions useful in delivering a variety of nucleic acid segments, including those encoding therapeutic polypeptides to selected mammalian host cells for use in therapeutic autoimmune modalities, including, for example, the *in vivo* induction of immunological tolerance *via* a liver-directed AAV-based gene therapeutic regimen for treating and/or ameliorating autoimmune disorders such as multiple sclerosis.

## Description

### CROSS-REFERENCE TO RELATED APPLICATIONS

This application claims the benefit under 35 U.S.C. § 119(e) of U.S. provisional application number 61/983,924, filed April 24, 2014, the contents of which are incorporated by reference herein in their entirety.

### FIELD OF THE INVENTION

The present invention relates generally to the fields of molecular biology and virology, and in particular, to the development of gene therapy vectors and methods for treatment of autoimmune diseases, such as multiple sclerosis (MS).

### BACKGROUND OF THE INVENTION

***Multiple Sclerosis (MS).*** MS is a multifocal demyelinating disease with progressive neurodegeneration caused by an autoimmune response to self-antigens in a genetically susceptible individual. Depending on where in the CNS the damage occurs, symptoms may include problems with muscle control, balance, vision, or speech. 250,000 to 350,000 people in the US. MS is an autoimmune disease that develops (in part) from a failure of central and peripheral tolerance mechanisms (particularly Tregs) to maintain self-tolerance and control potentially pathogenic autoreactive lymphocytes.^{2,3} It is characterized by chronic lymphocyte infiltration and inflammation of the CNS resulting in demyelination.

***Gene Therapy.*** Major advances in the field of gene therapy have been achieved by using viruses to deliver therapeutic genetic material. The adeno-associated virus (AAV) has attracted considerable attention as a highly effective viral vector for gene therapy due to its low immunogenicity and ability to effectively transduce non-dividing cells. AAV has been shown to infect a variety of cell and tissue types, and significant progress has been made over the last decade to adapt this viral system for use in human gene therapy.

In its normal "wild type" form, AAV DNA is packaged into the viral capsid as a single-stranded molecule about 4600 nucleotides (nt) in length. Following infection of the cell by the virus, the molecular machinery of the cell converts the single-stranded DNA into a double-stranded form. Only this double-stranded DNA form can be transcribed by cellular enzymes into RNA, which is then translated into polypeptides by additional cellular pathways.

### BRIEF SUMMARY OF THE INVENTION

What is specifically lacking in the prior art, however, are viral vector-based gene therapy methods for treating and/or ameliorating one or more of symptoms of autoimmune diseases, including, for example, MS. The development of such vectors, and compositions comprising them would provide a major advancement in medicine, and particularly in the development of a gene therapy-based treatment modality for MS.

The present invention overcomes these and other limitations inherent in the prior art by providing, in part, novel AAV nucleic acid vectors that are capable of, and optimized for, liver-directed expression of full-length proteins such as neuroproteins (including, without limitation, MOG, PLP, and MBP) or functional fragments thereof, thus abrogating the need for identifying HLA-MHC-specific epitopes required for inducing antigen-specific Tregs. In some embodiments, the invention permits each patient undergoing treatment to generate his/her own unique antigen-specific Tregs, which makes the treatment more universally applicable and more clinically feasible than existing technologies.

2 million people worldwide are living with MS. Diagnosis generally occurs at ages of 20 to 40, but documented cases of MS in children as young as two have been reported. Schilder's disease is a rare progressive demyelinating disorder that usually begins in childhood. While there is currently no cure for MS, there are various MS treatment options which have shown a decrease in the severity and frequency of relapses and a delay in disease progression in numerous studies. Therapies with predominantly immunomodulating properties include e.g., Beta-interferons and Glatiramer acetate. Therapies with predominantly immunosuppressive properties include e.g., Natalizumab, Fingolimod, and Mitoxantrone. The development of protocols that stimulates Treg numbers and/or their function has become a significant focus in treating autoimmune diseases. In fact, many of the beneficial effects of currently FDA approved immune-modulators used in the treatment of MS are associated with restored Treg homeostasis.^{1,4,5}

AAV gene therapy has been proven to be a powerful new tool for the treatment of a broad spectrum of diseases, including restoration of vision in patients with Leber's congenital amaurosis by retinal gene transfer, and hemophilia B by hepatic gene therapy.^{6,7} According to aspects of the disclosure, it has been demonstrated that hepatic gene therapy transfer with AAV vectors can reliably induce a robust antigen-specific immune tolerance in experimental animals to a variety of proteins even when the antigen is subsequently expressed in a highly immunogenic manner in other organs. Together these results demonstrate that liver directed gene therapy can abrogate potential cytotoxic CD8⁺ T cell responses.^{1,8-13} Importantly, it has also been shown that this protocol can even eliminate pre-existing antibodies.¹ This finding is quite significant since there is an increasing body of evidence that B cells and autoantibodies may play a pathogenic role in demyelinating disease.^{14,15} Others have shown that transgenic mice or transient transfection by plasmid or Ad-vectors expressing myelin basic protein could prevent the onset of EAE disease in mice.^{16,17} Suppression was dependent on hepatic gene expression and was mediated by induction of Ag-specific Tregs. In contrast, aspects of the disclosure relate to treatment of certain autoimmune conditions, e.g., MS, utilizing AAV delivery of nucleic acids encoding one or more host proteins to the liver.

Hepatocyte-restricted transgene expression from an optimized AAV vector can reliably induce immune tolerance to various therapeutic proteins (e.g., mediated by Ag-specific CD4⁺CD25⁺FoxP3⁺ Tregs). The process suppresses antibody formation and cytotoxic CD8⁺ T cell responses against the transgene product. Hepatic transgene expression is maintained even when the antigen was subsequently expressed in a highly immunogenic manner in other organs. The process efficiently and rapidly reverses pre-existing high antibody titers, and provided long-term correction of haemostasis in a murine hemophilia B model. Importantly, the method does not require protein to be secreted to be functional.

In some embodiments, advantageously, the novel rAAV nucleic acid vectors, express constructs, and infectious virions and viral particles comprising them as disclosed herein preferably have an improved efficiency in transducing one or more mammlalian liver cells to provide persistent expression of one or more genes of interest.

The improved rAAV nucleic acid vectors provided hererin preferably transduce mammalian cells with sufficient transduction efficiency to suppress the immune response associated with MS in patients, and thus abrogate CNS inflammation, and immune-mediated damage that occurs in MS patients. Unlike current therapies, this gene-therapy based approach represents a persistent, long-term treatment that reduces the clinical disability experienced by MS patients.

In some embodiments, the invention provides improved rAAV particles including those derived from one or more serotypes as known in the art (including, for example, those selected from the group consisting of AAV1, AAV2, AAV3, AAV4, AAV5, AAV6, AAV7, AAV8, AAV9, AAV10, AAV11, and AAV12).

The present invention also concerns rAAV nucleic acid vectors, wherein the nucleic acid segment further comprises a promoter, an enhancer, a post-transcriptional regulatory sequence, a polyadenylation signal, or any combination thereof, operably linked to the nucleic acid segment that encodes the selected polynucleotide of interest.

In certain embodiments, the nucleic acid segments cloned into the novel rAAV expression vectors described herein will express or encode one or more polypeptides, peptides, ribozymes, peptide nucleic acids, siRNA's, RNAi's, antisense oligonucleotides, antisense polynucleotides, antibodies, antigen binding fragments, or any combination thereof.

As noted herein, the therapeutic agents useful in the invention may include one or more agonists, antagonists, anti-apoptosis factors, inhibitors, receptors, cytokines, cytotoxins, erythropoietic agents, glycoproteins, growth factors, growth factor receptors, hormones, hormone receptors, interferons, interleukins, interleukin receptors, nerve growth factors, neuroactive peptides, neuroactive peptide receptors, proteases, protease inhibitors, protein decarboxylases, protein kinases, protein kinase inhibitors, enzymes, receptor binding proteins, transport proteins or one or more inhibitors thereof, serotonin receptors, or one or more uptake inhibitors thereof, serpins, serpin receptors, tumor suppressors, diagnostic molecules, chemotherapeutic agents, cytotoxins, or any combination thereof.

The rAAV nucleic acid vectors of the present invention may be comprised within a virion or viral particle having a serotype that is selected from the group consisting of AAV serotype 1 (AAV1), AAV serotype 2 (AAV2), AAV serotype 3 (AAV3), AAV serotype 4 (AAV4), AAV serotype 5 (AAV5), AAV serotype 6 (AAV6), AAV serotype 7 (AAV7), AAV serotype 8 (AAV8), AAV serotype 9 (AAV9), AAV serotype 10 (AAV10), AAV serotype 11 (AAV11), or AAV serotype 12 (AAV12), or any other serotype as known to one of ordinary skill in the viral arts.

In related embodiments, the invention further provides populations and pluralities of rAAV nucleic acid vectors, virions, infectious viral particles, or host cells that include one or more nucleic acid segments that encode an autoimmune disease therapeutic agent.

The invention further provides compositions and formulations that include one or more of the proteins, nucleic acid segments, viral vectors, host cells, or viral particles of the present invention together with one or more pharmaceutically-acceptable buffers, diluents, or excipients. Such compositions may be included in one or more diagnostic or therapeutic kits, for diagnosing, preventing, treating or ameliorating one or more symptoms of a mammalian inflammatory disease, such as autoimmune disease, and in particular, for delivery of a therapeutic agent for the treatment of MS in a human.

The invention further includes a method for providing a mammal in need thereof with a diagnostically- or therapeutically-effective amount of a selected therapeutic agent, the method comprising administering to a cell, tissue or organ of a mammal in need thereof, an amount of one or more of the disclosed rAAV nucleic acid vectors; and for a time effective to provide the mammal with a diagnostically- or a therapeutically-effective amount of the selected therapeutic agent.

The invention further provides a method for diagnosing, preventing, treating, or ameliorating at least one or more symptoms of a disease, a disorder, a dysfunction, an injury, an abnormal condition, or trauma in a mammal. In an overall and general sense, the method includes at least the step of administering to a mammal in need thereof one or more of the disclosed rAAV nucleic acid vectors, in an amount and for a time sufficient to diagnose, prevent, treat or ameliorate the one or more symptoms of the disease, disorder, dysfunction, injury, abnormal condition, or trauma in the mammal.

The invention also provides a method of transducing a population of mammalian cells. In an overall and general sense, the method includes at least the step of introducing into one or more cells of the population, a composition that comprises an effective amount of one or more of the rAAV nucleic acid vectors disclosed herein.

In some embodiments, the invention also provides isolated nucleic acid segments that encode one or more of the rAAV vector-based gene therapy constructs as described herein, and provides recombinant vectors, virus particles, infectious virions, and isolated host cells that comprise one or more of the rAAV nucleic acid vectors described herein.

Additionally, the present invention provides compositions, as well as therapeutic and/or diagnostic kits that include one or more of the disclosed AAV nucleic acid vector or AAV particle compositions, formulated with one or more additional ingredients, or prepared with one or more instructions for their use.

In one aspect, the invention provides compositions comprising recombinant adeno-associated viral (AAV) nucleic acid vectors, virions, viral particles, and pharmaceutical formulations thereof, useful in methods for delivering genetic material encoding one or more beneficial or therapeutic product(s) to mammalian cells and tissues. In some embodiments, the compositions and methods of the invention provide a significant advancement in the art through their use in the treatment, prevention, and/or amelioration of symptoms of one or more mammalian inflammatory diseases, including autoimmune diseases such as MS and the like.

In some embodiments, the invention provides rAAV-based expression constructs that encode one or more mammalian therapeutic agent(s) (including, but not limited to, for example, protein(s), polypeptide(s), peptide(s), enzyme(s), antibodies, antigen binding fragments, as well as variants, and/or active fragments thereof), for use in the treatment, prophylaxis, and/or amelioration of one or more symptoms of a mammalian disease, dysfunction, injury, and/or disorder.

The improved nucleic acid vectors and expression systems of the present invention may also optionally further include a polynucleotide that comprises, consists essentially of, or consists of, one or more polylinkers, restriction sites, and/or multiple cloning region(s) to facilitate insertion (cloning) of one or more selected genetic elements, genes of interest, or therapeutic or diagnostic constructs into the rAAV vector at a selected site within the vector.

In further aspects of the present invention, the exogenous polynucleotide(s) that may be delivered into suitable host cells by the rAAV nucleic acid vectors disclosed herein are preferably of mammalian origin, with polynucleotides encoding one or more polypeptides or peptides of human, non-human primate, porcine, bovine, ovine, feline, canine, equine, epine, caprine, or lupine origin being particularly preferred.

The exogenous polynucleotide(s) that may be delivered into host cells by the disclosed viral nucleic acid vectors may, in certain embodiments, encode one or more proteins, one or more polypeptides, one or more peptides, one or more enzymes, or one or more antibodies (or antigen-binding fragments thereof), or alternatively, may express one or more siRNAs, ribozymes, antisense oligonucleotides, PNA molecules, or any combination thereof. When combinational gene therapies are desired, two or more different molecules may be produced from a single rAAV expression system, or alternatively, a selected host cell may be transfected with two or more unique rAAV expression systems, each of which may comprise one or more distinct polynucleotides that encode a therapeutic agent.

In other embodiments, the invention also provides rAAV nucleic acid vectors that are comprised within an infectious adeno-associated viral particle or a virion, as well as pluralities of such virions or infectious particles. Such vectors, particles, and virions may be comprised within one or more diluents, buffers, physiological solutions or pharmaceutical vehicles, or formulated for administration to a mammal in one or more diagnostic, therapeutic, and/or prophylactic regimens. The vectors, virus particles, virions, and pluralities thereof of the present invention may also be provided in excipient formulations that are acceptable for veterinary administration to selected livestock, exotics, domesticated animals, and companion animals (including pets and such like), as well as to non-human primates, zoological or otherwise captive specimens, and such like.

The invention also concerns host cells that comprise at least one of the disclosed rAAV nucleic acid expression vectors, or one or more virus particles or virions that comprise such an expression vector. Such host cells are particularly mammalian host cells, with human liver cells being particularly highly preferred, and may be either isolated, in cell or tissue culture. In the case of genetically modified animal models, the transformed host cells may even be comprised within the body of a non-human animal itself.

Compositions comprising one or more of the disclosed rAAV nucleic acid vectors, expression systems, infectious rAAV particles, or host cells also form part of the present invention, and particularly those compositions that further comprise at least a first pharmaceutically-acceptable excipient for use in therapy, and for use in the manufacture of medicaments for the treatment of one or more mammalian inflammatory diseases, disorders, dysfunctions, or trauma. Such pharmaceutical compositions may optionally further comprise one or more diluents, buffers, liposomes, a lipid, a lipid complex. Alternatively, the rAAV nucleic acid vectors or rAAV particles of the present invention may be comprised within a plurality of microspheres, nanoparticles, liposomes, or any combination thereof.

Kits comprising one or more of the disclosed rAAV nucleic acid vectors (as well as one or more virions, viral particles, transformed host cells or pharmaceutical compositions comprising such vectors, virons, particle, or host cells); and instructions for using such kits in one or more therapeutic, diagnostic, and/or prophylactic clinical embodiments are also provided by the present invention. Such kits may further comprise one or more reagents, restriction enzymes, peptides, therapeutics, pharmaceutical compounds, or means for delivery of the composition(s) to host cells, or to an animal (e.g., syringes, injectables, and the like). Exemplary kits include those for treating, preventing, or ameliorating the symptoms of a disease, deficiency, dysfunction, and/or injury, or may include components for the large-scale production of the viral vectors themselves, such as for commercial sale, or for use by others, including e.g., virologists, medical professionals, and the like.

Another important aspect of the present invention concerns methods of use of the disclosed rAAV nucleic acid vectors, virions, expression systems, compositions, and host cells described herein in the preparation of medicaments for diagnosing, preventing, treating or ameliorating at least one or more symptoms of a disease, a dysfunction, a disorder, an abnormal condition, a deficiency, injury, or trauma in an animal, and in particular, one or more autoimmune diseases in humans.

Compositions comprising one or more of the disclosed rAAV nucleic acid vectors, expression systems, infectious rAAV particles, and host cells also form part of the present invention, and particularly those compositions that further comprise at least a first pharmaceutically-acceptable excipient for use in the manufacture of medicaments and methods involving therapeutic administration of such rAAV nucleic vectors, rAAV particles, and host cells.

Another important aspect of the present invention concerns methods of use of the disclosed nucleic acid vectors, virions, expression systems, compositions, and host cells described herein in the preparation of medicaments for treating or ameliorating the symptoms of various autoimmune diseases, such as MS, in a mammal, and in particular one or more such diseases in a human.

In some embodiments of any one of the method provided, the method further comprises administering an mTOR inhibitor, e.g., rapamycin.

### BRIEF DESCRIPTION OF THE DRAWINGS

For promoting an understanding of the principles of the invention, reference will now be made to the embodiments, or examples, illustrated in the drawings and specific language will be used to describe the same. It will, nevertheless be understood that no limitation of the scope of the invention is thereby intended. Any alterations and further modifications in the described embodiments, and any further applications of the principles of the invention as described herein are contemplated as would normally occur to one of ordinary skill in the art to which the invention relates.

The following drawings form part of the present specification and are included to demonstrate certain aspects of the present invention. The invention may be better understood by reference to the following description taken in conjunction with the accompanying drawings, in which like reference numerals identify like elements, and in which:
**FIG.** 1 describes aspects of an experimental autoimmune encephalomyelitis murine model employed in the present study as an animal model for MS;
**FIG. 2A** **and** **FIG. 2B** show a mouse model and mean clinical score criteria for the EAE study;
**FIG.** 3 shows a comparison of exemplary methods of the present invention as contrasted with the cell-based delivery methods of the prior art;
**FIG. 4A and FIG. 4B** show the AAV8 expression of MOG. FIG. 4A shows the Western blot analysis from protein extracted from liver, while FIG. 4B shows the analysis of transcriptional levels using real-time RT-PCR;
**FIG. 5A** shows the mean clinical score of EAE mice. Five female mice were injected s.c. with Ag/CFA emulsion. Mean clinical score (±SEM) was recorded starting at day 12;
**FIG. 5B** shows the mean clinical score of EAE mice. Five female C57BL/6 mice were injected s.c. with MOG/CFA emulsion. Mean clinical score (±SEM) was recorded;
**FIG. 6A, FIG. 6B****, and** **FIG. 6C** show AAV8-MOG prevented development of EAE in C57BL/6 mice. C57BL/6 mice (n=5) were injected with AAV8-MOG or control. EAE was induced 2 weeks later. **FIG. 6A****:** Mean clinical score, **FIG. 6B****:** anti-MOG IgG1, **FIG. 6C****:** anti-MOG IgG2c;
**FIG. 7A****,** **FIG. 7B, and FIG. 7C** show that AAV8-vectored gene therapy prevents the onset of EAE in the animal model of MS;
**FIG. 8** shows that AAV8-MOG ameliorated the disease in the animal model of MS;

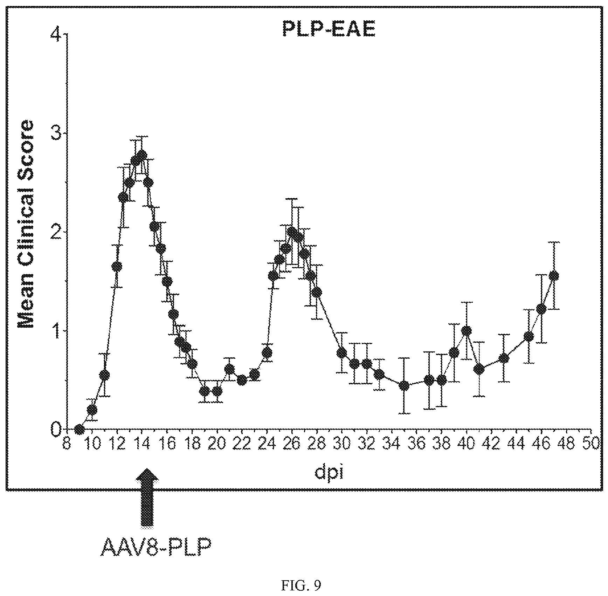

**FIG. 9** shows a PLP induced EAE naive control group to demonstrate disease progression;
**FIG. 10** shows effective suppression of pre-existing disease using the AAV8-vectored MOG treatment;
**FIG. 11** shows hepatic transgene expression of MOG. Western blot analysis from protein extracted from liver of MOG induced EAE mice injected with AAV8-MOG;
**FIG. 12** shows Luxol Fast Blue (LFB) staining of spinal cords from mice that received AAV8-GFP and had EAE induced (A) or not (B);
**FIG. 13A** shows mean clinical score (MCS) in EAE-induced C57BL/6 mice that received AAV8-MOG or control vector after the mice reached a MCS of about 0.3. Bar graphs show statistical significance between final scores and peak-to-final scores;
**FIG. 13B** shows mean clinical score (MCS) in EAE-induced C57BL/6 mice that received AAV8-MOG or control vector after the mice reached a MCS of about 0.8. Bar graphs show statistical significance between final scores and peak-to-final scores;
**FIG. 13C** shows mean clinical score (MCS) in EAE-induced C57BL/6 mice that received AAV8-MOG or control vector after the mice reached a MCS of about 1.3. Bar graphs show statistical significance between final scores and peak-to-final scores;
**FIG. 14A and 14B** shows serial sections of spinal cord from an EAE induced female mouse ∼35 days after receiving control vector (MCS=4.0). FIG. 14A is a hematoxylin and eosin stain showing areas of high inflammatory infiltration. FIG. 14B is a Luxol fast blue stain showing areas of demyelination. Circled areas highlight the co-localization of inflammation and loss of myelin;
**FIG. 15A and 15B** shows serial sections of spinal cord from an EAE induced female mouse ∼35 days after receiving AAV-MOG vector. (MCS =1.25) FIG. 15A is a hematoxylin and eosin stain showing diminished infiltration. FIG. 15B is a Luxol fast blue stain which appears to show that the section has less areas of demyelination as a result of the suppression of the inflammation;
**FIG. 16A** **and** **16B** show that Tregs isolated from spleens of AAV-MOG treated mice are functionally suppressive;
**FIG. 17A, 17B, 17C and 17D** show that AAV-MOG vector induces antigen specific Tregs. Splenocytes from mice injected with AAV-MOG vector 8 wks prior showed an increase in frequencies of I-Ab MOG₃₅₋₅₅ Tetramer positive CD4+ (FIG. 17A) and Treg+ (FIG. 17C) compared to control tetramer positive CD+ (FIG. 17B) and Treg+ (FIG. 17D).
**FIG. 18** shows that AAV8-PLP reduces clinical severity in mice with PLP induced relapsing-remitting EAE;
**FIG. 19A** shows aWestern blot analysis from protein extracted from liver of mice injected with AAV-MBP; and
**FIG. 19B** shows analysis of transcriptional levels of RNA obtained from the liver of mice treated with AAV-MBP or control by real-time RT-PCR.

### DETAILED DESCRIPTION

Recombinant adeno-associated virus (AAV) vectors have been used successfully for *in vivo* gene transfer in numerous pre-clinical animal models of human disease, and have been used successfully for long-term expression of a wide variety of therapeutic genes (Daya and Berns, 2008; Niemeyer *et al.,* 2009; Owen *et al.,* 2002; Keen-Rhinehart *et al.,* 2005; Scallan *et al.,* 2003; Song *et al.,* 2004). AAV vectors have also generated long-term clinical benefit in humans when targeted to immune-privileged sites, e.g., ocular delivery for Leber's congenital amaurosis (Bainbridge *et al.,* 2008; Maguire *et al.,* 2008; Cideciyan *et al.,* 2008). A major advantage of this vector is its comparatively low immune profile, eliciting only limited inflammatory responses and, in some cases, even directing immune tolerance to transgene products (LoDuca *et al.,* 2009). Nonetheless, the therapeutic efficiency, when targeted to non-immune privileged organs, has been limited in humans due to antibody and CD8⁺ T cell responses against the viral capsid, while in animal models, adaptive responses to the transgene product have also been reported (Manno *et al.,* 2006; Mingozzi *et al.,* 2007; Muruve *et al.,* 2008; Vandenberghe and Wilson, 2007; Mingozzi and High, 2007).

In some embodiments, a rAAV nucleic acid vector described herein comprises inverted terminal repeat sequences (ITRs), such as those derived from a wild-type AAV genome, such as the AAV2 genome. In some embodiments, the rAAV nucleic acid vector further comprises nucleic acid segment that includes a transgene (also referred to as a heterologous nucleic acid molecule) operably linked to a promoter and optionally, other regulatory elements, wherein the ITRs flank the nucleic acid segment.

In some embodiments, the promoter is a mammalian cell-specific or a mammalian tissue-specific promoter. In some embodiments, the promoter is a promoter that is capable of expressing the nucleic acid segment in one or more cells of a mammalian liver, such as hepatocyte cells. Exemplary hepatocyte specific promoters and enhancer elements include, e.g., albumin, human α1-antitrypsin (hAAT), transthyretin (TTR), and apolipoprotein E (apoE) promoters or enhancer elements.

In some embodiments, the transgene encodes an autoimmune disease therapeutic molecule of interest, such as a mammalian myelin basis protein (MBP), proteolipid protein (PLP), or myelin oligodendrocyte glycoprotein (MOG). As used herein, an autoimmune therapeutic molecule includes any antigen (such as a protein, fragment thereof, or a peptide) that contributes to initiation and/or progression of an autoimmune disease. Exemplary autoimmune therapeutic molecules include myelin basis protein (MBP, e.g., for multiple sclerosis), proteolipid protein (PLP, e.g., for multiple sclerosis), myelin oligodendrocyte glycoprotein (MOG, e.g., for multiple sclerosis), myelin-associated glycoprotein (MAG, e.g., for Anti-MAG Peripheral Neuropathy), insulin (e.g., for type 1 diabetes), islet-specific glucose-6-phosphatase catalytic subunit-related protein (IGRP, e.g., for type 1 diabetes), Preproinsulin (e.g., for type 1 diabetes), Glutamic decarboxylase (GAD, e.g., for type 1 diabetes), tyrosine phosphatase like autoantigen (e.g., for type 1 diabetes), insulinoma antigen- 2 (e.g., for type 1 diabetes), Islet cell antigen (e.g., for type 1 diabetes); thyroid stimulating hormone (TSH) receptor (e.g., for Graves disease), thyrotropin receptor (e.g., for Graves disease), Aggrecan (e.g., for rheumatoid arthritis), CD4+T cell epitope (GRVRVNSAY (SEQ ID NO: 36), e.g., for proteoglycaneinduced arthritis (PGIA) or rheumatoid arthritis), or acetylcholine receptor (e.g., for Myasthenia gravis). In some embodiments, the autoimmune disease therapeutic molecule of interest is a human protein, such as human myelin basis protein (MBP), a human proteolipid protein (PLP), or a human myelin oligodendrocyte glycoprotein (MOG).

Exemplary transgene sequences (e.g., cDNA sequences) and protein sequences that may be encoded by the transgene are provided below. In some embodiments, the transgene comprises a sequence that is at least 80%, 85%, 90%, 91%, 92%, 93%, 94%, 95%, 96%, 97%, 98%, or 99% identical to any one of the cDNA sequences provided below (SEQ ID NOs: 8, 10, 12, 14, 16, 18, 20, 22, 24, 26, 28, 30, 32, or 34). In some embodiments, the transgene comprises a sequence that is any one of the cDNA sequences provided below (SEQ ID NOs: 8, 10, 12, 14, 16, 18, 20, 22, 24, 26, 28, 30, 32, or 34). In some embodiments, the transgene is codon-optimized for expression in human cells. In some embodiments, the transgene contains a nucleotide sequence that encodes at least 20, 30, 40, 50, 60, 70, 80, 90, 100, 110, 120, 130, 140, 150, 160, 170, 180, 190, 200, 210, 220, 230, 240 or more contiguous amino acids of any one of the protein sequences provided herein (e.g., any one of SEQ ID NOs: 1, 2, 3, 9, 11, 13, 15, 17, 19, 21, 23, 25, 27, 29, 31, 33, or 35). In some embodiments, the transgene contains a nucleotide sequence that encodes a protein that is at least 80%, 85%, 90%, 91%, 92%, 93%, 94%, 95%, 96%, 97%, 98%, or 99% identical to any one of the protein sequences provided herein (e.g., any one of SEQ ID NOs: 1, 2, 3, 9, 11, 13, 15, 17, 19, 21, 23, 25, 27, 29, 31, 33, or 35). In some embodiments, the transgene contains a nucleotide sequence that encodes any one of the protein sequences provided herein (e.g., any one of SEQ ID NOs: 1, 2, 3, 9, 11, 13, 15, 17, 19, 21, 23, 25, 27, 29, 31, 33, or 35).

Exemplary Mus musculus myelin oligodendrocyte glycoprotein (MOG) cDNA

1 atggcctgtt tgtggagctt ctctttgccc agctgcttcc tctcccttct cctcctcctt
61 ctcctccagt tgtcatgcag ctatgcagga caattcagag tgataggacc agggtatccc
121 atccgggctt tagttgggga tgaagcagag ctgccgtgcc gcatctctcc tgggaaaaat
181 gccacgggca tggaggtggg ttggtaccgt tctcccttct caagagtggt tcacctctac
241 cgaaatggca aggaccaaga tgcagagcaa gcacctgaat accggggacg cacagagctt
301 ctgaaagaga ctatcagtga gggaaaggtt acccttagga ttcagaacgt gagattctca
361 gatgaaggag gctacacctg cttcttcaga gaccactctt accaagaaga ggcagcaatg
421 gagttgaaag tggaagatcc cttctattgg gtcaaccccg gtgtgctgac tctcatcgca
481 cttgtgccta cgatcctcct gcaggtctct gtaggccttg tattcctctt cctgcagcac
541 agactgagag gaaaacttcg tgcagaagta gagaatctcc atcggacttt tgatcctcac
601 ttcctgaggg tgccctgctg gaagataaca ctgtttgtta ttgtgcctgt tcttggaccc
661 ctggttgcct tgatcatctg ctacaactgg ctgcaccgaa gactggcagg acagtttctt
721 gaagagctaa gaaacccct ttga (SEQ ID NO: 8)

Exemplary mus musculus myelin-oligodendrocyte glycoprotein (MOG) protein

1 maclwsfswp scflslllll llqlscsyag qfrvigpgyp iralvgdeae lpcrispgkn
61 atgmevgwyr spfsrvvhly rngkdqdaeq apeyrgrtel lketisegkv tlriqnvrfs
121 deggytcffr dhsyqeeaam elkvedpfyw vnpgvltlia lvptillqvs vglvflflqh
181 rlrgklraev enlhrtfdph flrvpcwkit lfvivpvlgp lvaliicynw lhrrlagqfl
241 eelrnpf (SEQ ID NO: 9)

Exemplary Mus musculus proteolipid protein 1 (PLP) cDNA

ATGGGCTTGTTAGAGTGTTGTGCTAGATGTCTGGTAGGGGCCCCCTTTGCTTCCCTGGTGGCCACTGGAT
TGTGTTTCTTTGGAGTGGCACTGTTCTGTGGATGTGGACATGAAGCTCTCACTGGTACAGAAAAGCTAAT
TGAGACCTATTTCTCCAAAAACTACCAGGACTATGAGTATCTCATTAATGTGATTCATGCTTTCCAGTAT
GTCATCTATGGAACTGCCTCTTTCTTCTTCCTTTATGGGGCCCTCCTGCTGGCTGAGGGCTTCTACACCA
CCGGCGCTGTCAGGCAGATCTTTGGCGACTACAAGACCACCATCTGCGGCAAGGGCCTGAGCGCAACGGT
AACAGGGGGCCAGAAGGGGAGGGGTTCCAGAGGCCAACATCAAGCTCATTCTTTGGAGCGGGTGTGTCAT
TGTTTGGGAAAATGGCTAGGACATCCCGACAAGTTTGTGGGCATCACCTATGCCCTGACTGTTGTATGGC
TCCTGGTGTTTGCCTGCTCGGCTGTACCTGTGTACATTTACTTCAATACCTGGACCACCTGTCAGTCTAT
TGCCTTCCCTAGCAAGACCTCTGCCAGTATAGGCAGTCTCTGCGCTGATGCCAGAATGTATGGTGTTCTC
CCATGGAATGCTTTCCCTGGCAAGGTTTGTGGCTCCAACCTTCTGTCCATCTGCAAAACAGCTGAGTTCC

AAATGACCTTCCACCTGTTTATTGCTGCGTTTGTGGGTGCTGCGGCCACACTAGTTTCCCTGCTCACCTT
CATGATTGCTGCCACTTACAACTTCGCCGTCCTTAAACTCATGGGCCGAGGCACCAAGTTCTGA (SEQ ID
NO: 10)

Exemplary Mus musculus proteolipid protein 1 (PLP) protein

1 mglleccarc lvgapfaslv atglcffgva lfcgcgheal tgtekliety fsknyqdyey
 61 linvihafqy viygtasfff lygalllaeg fyttgavrqi fgdyktticg kglsatvtgg
121 qkgrgsrgqh qahslervch clgkwlghpd kfvgityalt vvwllvfacs avpvyiyfnt
181 wttcqsiafp sktsasigsl cadarmygvl pwnafpgkvc gsnllsickt aefqmtfhlf
241 iaafvgaaat lvsllltfmia atynfavlkl mgrgtkf (SEQ ID NO: 11)

Exemplary Mus musculus myelin basic protein (MBP) cDNA

ATGGGAAACCACTCTGGAAAGAGAGAATTATCTGCTGAGAAGGCCAGTAAGGATGGAGAGATTCACCGAG
GAGAGGCTGGAAAGAAGAGAAGCGTGGGCAAGCTTTCTCAGACGGCCTCAGAGGACAGTGATGTGTTTGG
GGAGGCAGATGCGATCCAGAACAATGGGACCTCGGCTGAGGACACGGCGGTGACAGACTCCAAGCACACA
GCAGACCCAAAGAATAACTGGCAAGGCGCCCACCCAGCTGACCCAGGGAACCGCCCCCACTTGATCCGCC
TCTTTTCCCGAGATGCCCCGGGAAGGGAGGACAACACCTTCAAAGACAGGCCCTCAGAGTCCGACGAGCT
TCAGACCATCCAAGAAGACCCCACAGCAGCTTCCGGAGGCCTGGATGTGATGGCATCACAGAAGAGACCC
TCACAGCGATCCAAGTACCTGGCCACAGCAAGTACCATGGACCATGCCAGGCATGGCTTCCTCCCAAGGC
ACAGAGACACGGGCATCCTTGACTCCATCGGGCGCTTCTTTAGCGGTGACAGGGGTGCGCCCAAGCGGGG
CTCTGGCAAGGTGAGCTCCGAGCCGTAG  (SEQ ID NO: 12)

Exemplary Mus musculus myelin basic protein (MBP) protein

1 mgnhsgkrel saekaskdge ihrgeagkkr svgklsqtas edsdvfgead aiqnngtsae
61 dtavtdskht adpknnwqga hpadpgnrph lirlfsrdap gredntfkdr psesdelqti
121 qedptaasgg ldvmasqkrp sqrskylata stmdharhgf lprhrdtgil dsigrffsgd
181 rgapkrgsgk vssep (SEQ ID NO: 13)

Exemplary Homo sapiens myelin oligodendrocyte glycoprotein (MOG) cDNA

1 atggcaagct tatcgagacc ctctctgccc agctgcctct gctccttcct cctcctcctc
61 ctcctccaag tgtcttccag ctatgcaggg cagttcagag tgataggacc aagacaccct
121 atccgggctc tggtcgggga tgaagtggaa ttgccatgtc gcatatctcc tgggaagaac
181 gctacaggca tggaggtggg gtggtaccgc ccccccttct ctagggtggt tcatctctac
241 agaaatggca aggaccaaga tggagaccag gcacctgaat atcggggccg gacagagctg
301 ctgaaagatg ctattggtga gggaaaggtg actctcagga tccggaatgt aaggttctca
361 gatgaaggag gtttcacctg cttcttccga gatcattctt accaagagga ggcagcaatg
421 gaattgaaag tagaagatcc tttctactgg gtgagccctg gagtgctggt tctcctcgcg
481 gtgctgcctg tgctcctcct gcagatcact gttggcctcg tcttcctctg cctgcagtac
541 agactgagag gaaaacttcg agcagagata gagaatctcc accggacttt tgatccccac
601 tttctgaggg tgccctgctg gaagataacc ctgtttgtaa ttgtgccggt tcttggaccc
661 ttggttgcct tgatcatctg ctacaactgg ctacatcgaa gactagcagg gcaattcctt
721 gaagagctac gaaatccctt ctga (SEQ ID NO: 14)

Exemplary Homo sapiens myelin oligodendrocyte glycoprotein (MOG) protein

1 maslsrpslp sclcsfllll llqvsssyag qfrvigprhp iralvgdeve lpcrispgkn
61 atgmevgwyr ppfsrvvhly rngkdqdgdq apeyrgrtel lkdaigegkv tlrirnvrfs
121 deggftcffr dhsyqeeaam elkvedpfyw vspgvlvlla vlpvlllqit vglvflclqy
181 rlrgklraei enlhrtfdph flrvpcwkit lfvivpvlgp lvaliicynw lhrrlagqfl
241 eelrnpf (SEQ ID NO: 15)

Exemplary Homo sapiens myelin basic protein (MBP), transcript variant 7, cDNA

ATGGGAAACCACGCAGGCAAACGAGAATTAAATGCCGAGAAGGCCAGTACGAATAGTGAAACTAACAGAG
GAGAATCTGAAAAAAAGAGAAACCTGGGTGAACTTTCACGGACAACCTCAGAGGACAACGAAGTGTTCGG
AGAGGCAGATGCGAACCAGAACAATGGGACCTCCTCTCAGGACACAGCGGTGACTGACTCCAAGCGCACA
GCGGACCCGAAGAATGCCTGGCAGGATGCCCACCCAGCTGACCCAGGGAGCCGCCCCCACTTGATCCGCC
TCTTTTCCCGAGATGCCCCGGGAGGGAGGACAACACCTTCAAAGACAGGCCCTCTGAGTCCGACGAGCT
CCAGACCATCCAAGAAGACAGTGCAGCCACCTCCGAGAGCCTGGATGTGATGGCGTCACAGAAGAGACCC
TCCCAGAGGCACGGATCCAAGTACCTGGCCACAGCAAGTACCATGGACCATGCCAGGCATGGCTTCCTCC
CAAGGCACAGAGACACGGGCATCCTTGACTCCATCGGGCGCTTCTTTGGCGGTGACAGGGGTGCGCCCAA
GCGGGGCTCTGGCAAGGACTCACACCACCCGGCAAGAACTGCTCACTACGGCTCCCTGCCCCAGAAGTCA
CACGGCCGGACCCAAGATGAAAACCCCGTAGTCCACTTCTTCAAGAACATTGTGACGCCTCGCACACCAC
CCCCGTCGCAGGGAAAGGGGAGAGGACTGTCCCTGAGCAGATTTAGCTGGGGGGCCGAAGGCCAGAGACC
AGGATTTGGCTACGGAGGCAGAGCGTCCGACTATAAATCGGCTCACAAGGGATTCAAGGGAGTCGATGCC
CAGGGCACGCTTTCCAAAATTTTTAAGCTGGGAGGAAGAGATAGTCGCTCTGGATCACCCATGGCTAGAC
GCTGA (SEQ ID NO: 16)

Exemplary Homo sapiens myelin basic protein (MBP), transcript variant 7, protein

1 mgnhagkrel naekastnse tnrgesekkr nlgelsrtts ednevfgead anqnngtssq
61 dtavtdskrt adpknawqda hpadpgsrph lirlfsrdap gredntfkdr psesdelqti
121 qedsaatses ldvmasqkrp sqrhgskyla tastmdharh gflprhrdtg ildsigrffg
181 gdrgapkrgs gkdshhpart ahygslpqks hgrtqdenpv vhffknivtp rtpppsqgkg
241 rglslsrfsw gaegqrpgfg yggrasdyks ahkgfkgvda qgtlskifkl ggrdsrsgsp
301 marr (SEQ ID NO: 17)

Exemplary Homo sapiens myelin basic protein (MBP), transcript variant 1, cDNA

ATGGCGTCACAGAAGAGACCCTCCCAGAGGCACGGATCCAAGTACCTGGCCACAGCAAGTACCATGGACC
ATGCCAGGCATGGCTTCCTCCCAAGGCACAGAGACACGGGCATCCTTGACTCCATCGGGCGCTTCTTTGG
CGGTGACAGGGGTGCGCCCAAGCGGGGCTCTGGCAAGGTACCCTGGCTAAAGCCGGGCCGGAGCCCTCTG
CCCTCTCATGCCCGCAGCCAGCCTGGGCTGTGCAACATGTACAAGGACTCACACCACCCGGCAAGAACTG
CTCACTACGGCTCCCTGCCCCAGAAGTCACACGGCCGGACCCAAGATGAAAACCCCGTAGTCCACTTCTT
CAAGAACATTGTGACGCCTCGCACACCACCCCCGTCGCAGGGAAAGGGGAGAGGACTGTCCCTGAGCAGA
TTTAGCTGGGGGGCCGAAGGCCAGAGACCAGGATTTGGCTACGGAGGCAGAGCGTCCGACTATAAATCGG
CTCACAAGGGATTCAAGGGAGTCGATGCCCAGGGCACGCTTTCCAAAATTTTTAAGCTGGGAGGAAGAGA
TAGTCGCTCTGGATCACCCATGGCTAGACGCTGA (SEQ ID NO: 18)

Exemplary Homo sapiens myelin basic protein (MBP), transcript variant 1, protein

1 masqkrpsqr hgskylatas tmdharhgfl prhrdtgild sigrffggdr gapkrgsgkv
61 pwlkpgrspl psharsqpgl cnmykdshhp artahygslp qkshgrtqde npvvhffkni
121 vtprtpppsq gkgrglslsr fswgaegqrp gfgyggrasd yksahkgfkg vdaqgtlski
181 fklggrdsrs gspmarr (SEQ ID NO: 19)

Exemplary Homo sapiens myelin basic protein (MBP), transcript variant 2, cDNA

ATGGCGTCACAGAAGAGACCCTCCCAGAGGCACGGATCCAAGTACCTGGCCACAGCAAGTACCATGGACC
ATGCCAGGCATGGCTTCCTCCCAAGGCACAGAGACACGGGCATCCTTGACTCCATCGGGCGCTTCTTTGG
CGGTGACAGGGGTGCGCCCAAGCGGGGCTCTGGCAAGGTACCCTGGCTAAAGCCGGGCCGGAGCCCTCTG
CCCTCTCATGCCCGCAGCCAGCCTGGGCTGTGCAACATGTACAAGGACTCACACCACCCGGCAAGAACTG
CTCACTACGGCTCCCTGCCCCAGAAGTCACACGGCCGGACCCAAGATGAAAACCCCGTAGTCCACTTCTT
CAAGAACATTGTGACGCCTCGCACACCACCCCCGTCGCAGGGAAAGGGGGCCGAAGGCCAGAGACCAGGA
TTTGGCTACGGAGGCAGAGCGTCCGACTATAAATCGGCTCACAAGGGATTCAAGGGAGTCGATGCCCAGG
GCACGCTTTCCAAAATTTTTAAGCTGGGAGGAAGAGATAGTCGCTCTGGATCACCCATGGCTAGACGCTG
A (SEQ ID NO: 20)

Exemplary Homo sapiens myelin basic protein (MBP), transcript variant 2, protein

1 masqkrpsqr hgskylatas tmdharhgfl prhrdtgild sigrffggdr gapkrgsgkv
61 pwlkpgrspl psharsqpgl cnmykdshhp artahygslp qkshgrtqde npvvhffkni
121 vtprtpppsq gkgaegqrpg fgyggrasdy ksahkgfkgv daqgtlskif klggrdsrsg
181 spmarr (SEQ ID NO: 21)

Exemplary Homo sapiens myelin basic protein (MBP), transcript variant 3, cDNA

ATGGCGTCACAGAAGAGACCCTCCCAGAGGCACGGATCCAAGTACCTGGCCACAGCAAGTACCATGGACC
ATGCCAGGCATGGCTTCCTCCCAAGGCACAGAGACACGGGCATCCTTGACTCCATCGGGCGCTTCTTTGG
CGGTGACAGGGGTGCGCCCAAGCGGGGCTCTGGCAAGGACTCACACCACCCGGCAAGAACTGCTCACTAC
GGCTCCCTGCCCCAGAAGTCACACGGCCGGACCCAAGATGAAAACCCCGTAGTCCACTTCTTCAAGAACA
TTGTGACGCCTCGCACACCACCCCCGTCGCAGGGAAAGGGGAGAGGACTGTCCCTGAGCAGATTTAGCTG
GGGGGCCGAAGGCCAGAGACCAGGATTTGGCTACGGAGGCAGAGCGTCCGACTATAAATCGGCTCACAAG
GGATTCAAGGGAGTCGATGCCCAGGGCACGCTTTCCAAAATTTTTAAGCTGGGAGGAAGAGATAGTCGCT
CTGGATCACCCATGGCTAGACGCTGA  (SEQ ID NO: 22)

Exemplary Homo sapiens myelin basic protein (MBP), transcript variant 3, protein

1 masqkrpsqr hgskylatas tmdharhgfl prhrdtgild sigrffggdr gapkrgsgkd
61 shhpartahy gslpqkshgr tqdenpvvhf fknivtprtp ppsqgkgrgl slsrfswgae
121 gqrpgfgygg rasdyksahk gfkgvdaqgt lskifklggr dsrsgspmar r (SEQ ID
NO: 23)

Exemplary Homo sapiens myelin basic protein (MBP), transcript variant 4, cDNA

ATGGCGTCACAGAAGAGACCCTCCCAGAGGCACGGATCCAAGTACCTGGCCACAGCAAGTACCATGGACC
ATGCCAGGCATGGCTTCCTCCCAAGGCACAGAGACACGGGCATCCTTGACTCCATCGGGCGCTTCTTTGG
CGGTGACAGGGGTGCGCCCAAGCGGGGCTCTGGCAAGGACTCACACCACCCGGCAAGAACTGCTCACTAC
GGCTCCCTGCCCCAGAAGTCACACGGCCGGACCCAAGATGAAAACCCCGTAGTCCACTTCTTCAAGAACA
TTGTGACGCCTCGCACACCACCCCCGTCGCAGGGAAAGGGGGCCGAAGGCCAGAGACCAGGATTTGGCTA
CGGAGGCAGAGCGTCCGACTATAAATCGGCTCACAAGGGATTCAAGGGAGTCGATGCCCAGGGCACGCTT
TCCAAAATTTTTAAGCTGGGAGGAAGAGATAGTCGCTCTGGATCACCCATGGCTAGACGCTGA (SEQ ID
NO: 24)

Exemplary Homo sapiens myelin basic protein (MBP), transcript variant 4, protein

  1 masqkrpsqr hgskylatas tmdharhgfl prhrdtgild sigrffggdr gapkrgsgkd
 61 shhpartahy gslpqkshgr tqdenpvvhf fknivtprtp ppsqgkgaeg qrpgfgyggr
121 asdyksahkg fkgvdaqgtl skifklggrd srsgspmarr (SEQ ID NO: 25)

Exemplary Homo sapiens myelin basic protein (MBP), transcript variant 8, cDNA

ATGGGAAACCACGCAGGCAAACGAGAATTAAATGCCGAGAAGGCCAGTACGAATAGTGAAACTAACAGAG
GAGAATCTGAAAAAAAGAGAAACCTGGGTGAACTTTCACGGACAACCTCAGAGGACAACGAAGTGTTCGG
AGAGGCAGATGCGAACCAGAACAATGGGACCTCCTCTCAGGACACAGCGGTGACTGACTCCAAGCGCACA
GCGGACCCGAAGAATGCCTGGCAGGATGCCCACCCAGCTGACCCAGGGAGCCGCCCCCACTTGATCCGCC
TCTTTTCCCGAGATGCCCCGGGGAGGGAGGACAACACCTTCAAAGACAGGCCCTCTGAGTCCGACGAGCT
CCAGACCATCCAAGAAGACAGTGCAGCCACCTCCGAGAGCCTGGATGTGATGGCGTCACAGAAGAGACCC
TCCCAGAGGCACGGATCCAAGTACCTGGCCACAGCAAGTACCATGGACCATGCCAGGCATGGCTTCCTCC
CAAGGCACAGAGACACGGGCATCCTTGACTCCATCGGGCGCTTCTTTGGCGGTGACAGGGGTGCGCCCAA
GCGGGGCTCTGGCAAGGTGAGCTCTGAGGAGTAG (SEQ ID NO: 26)

Exemplary Homo sapiens myelin basic protein (MBP), transcript variant 8, protein

1 mgnhagkrel naekastnse tnrgesekkr nlgelsrtts ednevfgead anqnngtssq
61 dtavtdskrt adpknawqda hpadpgsrph lirlfsrdap gredntfkdr psesdelqti

121 qedsaatses ldvmasqkrp sqrhgskyla tastmdharh gflprhrdtg ildsigrffg
181 gdrgapkrgs gkvssee (SEQ ID NO: 27)

Exemplary Homo sapiens proteolipid protein 1 (PLP1), transcript variant 1, cDNA

ATGGGCTTGTTAGAGTGCTGTGCAAGATGTCTGGTAGGGGCCCCCTTTGCTTCCCTGGTGGCCACTGGAT
TGTGTTTCTTTGGGGTGGCACTGTTCTGTGGCTGTGGACATGAAGCCCTCACTGGCACAGAAAAGCTAAT
TGAGACCTATTTCTCCAAAAACTACCAAGACTATGAGTATCTCATCAATGTGATCCATGCCTTCCAGTAT
GTCATCTATGGAACTGCCTCTTTCTTCTTCCTTTATGGGGCCCTCCTGCTGGCTGAGGGCTTCTACACCA
CCGGCGCAGTCAGGCAGATCTTTGGCGACTACAAGACCACCATCTGCGGCAAGGGCCTGAGCGCAACGGT
AACAGGGGGCCAGAAGGGGAGGGGTTCCAGAGGCCAACATCAAGCTCATTCTTTGGAGCGGGTGTGTCAT
TGTTTGGGAAAATGGCTAGGACATCCCGACAAGTTTGTGGGCATCACCTATGCCCTGACCGTTGTGTGGC
TCCTGGTGTTTGCCTGCTCTGCTGTGCCTGTGTACATTTACTTCAACACCTGGACCACCTGCCAGTCTAT
TGCCTTCCCCAGCAAGACCTCTGCCAGTATAGGCAGTCTCTGTGCTGATGCCAGAATGTATGGTGTTCTC
CCATGGAATGCTTTCCCTGGCAAGGTTTGTGGCTCCAACCTTCTGTCCATCTGCAAAACAGCTGAGTTCC
AAATGACCTTCCACCTGTTTATTGCTGCATTTGTGGGGGCTGCAGCTACACTGGTTTCCCTGCTCACCTT
CATGATTGCTGCCACTTACAACTTTGCCGTCCTTAAACTCATGGGCCGAGGCACCAAGTTCTGA (SEQ ID
NO: 28)

Exemplary Homo sapiens proteolipid protein 1 (PLP1), transcript variant 1, protein

1 mglleccarc lvgapfaslv atglcffgva lfcgcgheal tgtekliety fsknyqdyey
61 linvihafqy viygtasfff lygalllaeg fyttgavrqi fgdykttícg kglsatvtgg
121 qkgrgsrgqh qahslervch clgkwlghpd kfvgityalt vvwllvfacs avpvyiyfnt
181 wttcqsiafp sktsasigsl cadarmygvl pwnafpgkvc gsnllsickt aefqmtfhlf
241 iaafvgaaat lvsllltfmia atynfavlkl mgrgtkf (SEQ ID NO: 29)

Exemplary Homo sapiens proteolipid protein 1 (PLP1), transcript variant 2, cDNA

ATGGGCTTGTTAGAGTGCTGTGCAAGATGTCTGGTAGGGGCCCCCTTTGCTTCCCTGGTGGCCACTGGAT
TGTGTTTCTTTGGGGTGGCACTGTTCTGTGGCTGTGGACATGAAGCCCTCACTGGCACAGAAAAGCTAAT
TGAGACCTATTTCTCCAAAAACTACCAAGACTATGAGTATCTCATCAATGTGATCCATGCCTTCCAGTAT
GTCATCTATGGAACTGCCTCTTTCTTCTTCCTTTATGGGGCCCTCCTGCTGGCTGAGGGCTTCTACACCA
CCGGCGCAGTCAGGCAGATCTTTGGCGACTACAAGACCACCATCTGCGGCAAGGGCCTGAGCGCAACGTT
TGTGGGCATCACCTATGCCCTGACCGTTGTGTGGCTCCTGGTGTTTGCCTGCTCTGCTGTGCCTGTGTAC
ATTTACTTCAACACCTGGACCACCTGCCAGTCTATTGCCTTCCCCAGCAAGACCTCTGCCAGTATAGGCA
GTCTCTGTGCTGATGCCAGAATGTATGGTGTTCTCCCATGGAATGCTTTCCCTGGCAAGGTTTGTGGCTC
CAACCTTCTGTCCATCTGCAAAACAGCTGAGTTCCAAATGACCTTCCACCTGTTTATTGCTGCATTTGTG
GGGGCTGCAGCTACACTGGTTTCCCTGCTCACCTTCATGATTGCTGCCACTTACAACTTTGCCGTCCTTA
AACTCATGGGCCGAGGCACCAAGTTCTGA (SEQ ID NO: 30)

Exemplary Homo sapiens proteolipid protein 1 (PLP1), transcript variant 2, protein

1 mglleccarc lvgapfaslv atglcffgva lfcgcgheal tgtekliety fsknyqdyey
61 linvihafqy viygtasfff lygalllaeg fyttgavrqi fgdyktticg kglsatfvgi
121 tyaltvvwll vfacsavpvy iyfntwttcq siafpsktsa sigslcadar mygvlpwnaf
181 pgkvcgsnll sicktaefqm tfhlfiaafv gaaatlvsll tfmiaatynf avlklmgrgt
241 kf (SEQ ID NO: 31)

Exemplary Homo sapiens proteolipid protein 1 (PLP1), transcript variant 3, cDNA

ATGGGCTTGTTAGAGTGCTGTGCAAGATGTCTGGTAGGGGCCCCCTTTGCTTCCCTGGTGGCCACTGGAT
TGTGTTTCTTTGGGGTGGCACTGTTCTGTGGCTGTGGACATGAAGCCCTCACTGGCACAGAAAAGCTAAT
TGAGACCTATTTCTCCAAAAACTACCAAGACTATGAGTATCTCATCAATGTGATCCATGCCTTCCAGTAT
GTCATCTATGGAACTGCCTCTTTCTTCTTCCTTTATGGGGCCCTCCTGCTGGCTGAGGGCTTCTACACCA
CCGGCGCAGTCAGGCAGATCTTTGGCGACTACAAGACCACCATCTGCGGCAAGGGCCTGAGCGCAACGGT
AACAGGGGGCCAGAAGGGGAGGGGTTCCAGAGGCCAACATCAAGCTCATTCTTTGGAGCGGGTGTGTCAT
TGTTTGGGAAAATGGCTAGGACATCCCGACAAGTTTGTGGGCATCACCTATGCCCTGACCGTTGTGTGGC

TCCTGGTGTTTGCCTGCTCTGCTGTGCCTGTGTACATTTACTTCAACACCTGGACCACCTGCCAGTCTAT
TGCCTTCCCCAGCAAGACCTCTGCCAGTATAGGCAGTCTCTGTGCTGATGCCAGAATGTATGGTGTTCTC
CCATGGAATGCTTTCCCTGGCAAGGTTTGTGGCTCCAACCTTCTGTCCATCTGCAAAACAGCTGAGTTCC
AAATGACCTTCCACCTGTTTATTGCTGCATTTGTGGGGGCTGCAGCTACACTGGTTTCCCTGCTCACCTT
CATGATTGCTGCCACTTACAACTTTGCCGTCCTTAAACTCATGGGCCGAGGCACCAAGTTCTGA (SEQ ID
NO: 32)

Exemplary Homo sapiens proteolipid protein 1 (PLP1), transcript variant 3, protein

1 mglleccarc lvgapfaslv atglcffgva lfcgcgheal tgtekliety fsknyqdyey
 61 linvihafqy viygtasfff lygalllaeg fyttgavrqi fgdykttícg kglsatvtgg
121 qkgrgsrgqh qahslervch clgkwlghpd kfvgityalt vvwllvfacs avpvyiyfnt
181 wttcqsiafp sktsasigsl cadarmygvl pwnafpgkvc gsnllsickt aefqmtfhlf
241 iaafvgaaat lvslltfmia atynfavlkl mgrgtkf (SEQ ID NO: 33)

Exemplary Homo sapiens proteolipid protein 1 (PLP1), transcript variant 4, cDNA

ATGGACTATGAGTATCTCATCAATGTGATCCATGCCTTCCAGTATGTCATCTATGGAACTGCCTCTTTCT
TCTTCCTTTATGGGGCCCTCCTGCTGGCTGAGGGCTTCTACACCACCGGCGCAGTCAGGCAGATCTTTGG
CGACTACAAGACCACCATCTGCGGCAAGGGCCTGAGCGCAACGGTAACAGGGGGCCAGAAGGGGAGGGGT
TCCAGAGGCCAACATCAAGCTCATTCTTTGGAGCGGGTGTGTCATTGTTTGGGAAAATGGCTAGGACATC
CCGACAAGTTTGTGGGCATCACCTATGCCCTGACCGTTGTGTGGCTCCTGGTGTTTGCCTGCTCTGCTGT
GCCTGTGTACATTTACTTCAACACCTGGACCACCTGCCAGTCTATTGCCTTCCCCAGCAAGACCTCTGCC
AGTATAGGCAGTCTCTGTGCTGATGCCAGAATGTATGGTGTTCTCCCATGGAATGCTTTCCCTGGCAAGG
TTTGTGGCTCCAACCTTCTGTCCATCTGCAAAACAGCTGAGTTCCAAATGACCTTCCACCTGTTTATTGC
TGCATTTGTGGGGGCTGCAGCTACACTGGTTTCCCTGCTCACCTTCATGATTGCTGCCACTTACAACTTT
GCCGTCCTTAAACTCATGGGCCGAGGCACCAAGTTCTGA (SEQ ID NO: 34)

Exemplary Homo sapiens proteolipid protein 1 (PLP1), transcript variant 4, protein

1 mdyeylinvi hafqyviygt asffflygal llaegfyttg avrqifgdyk tticgkglsa
61 tvtggqkgrg srgqhqahsl ervchclgkw lghpdkfvgi tyaltvvwll vfacsavpvy
121 iyfntwttcq siafpsktsa sigslcadar mygvlpwnaf pgkvcgsnll sicktaefqm
181 tfhlfiaafv gaaatlvsll tfmiaatynf avlklmgrgt kf (SEQ ID NO: 35)

In some embodiments, the rAAV nucleic acid vector is encapsidated by a rAAV particle as described herein. The rAAV particle may be of any AAV serotype (e.g., 1, 2, 3, 4, 5, 6, 7, 8, 9, or 10), including any derivative (including non-naturally occurring variants of a serotype) or pseudotype. In some embodiments, the rAAV particle is an AAV8 particle, which may be pseudotyped with AAV2 ITRs. Non-limiting examples of derivatives and pseudotypes include AAV2-AAV3 hybrid, AAVrh.10, AAVhu.14, AAV3a/3b, AAVrh32.33, AAV-HSC15, AAV-HSC17, AAVhu.37, AAVrh.8, CHt-P6, AAV2.5, AAV6.2, AAV2i8, AAV-HSC15/17, AAVM41, AAV9.45, AAV6(Y445F/Y731F), AAV2.5T, AAV-HAE1/2, AAV clone 32/83, AAVShH10, AAV2 (Y->F), AAV8 (Y733F), AAV2.15, AAV2.4, AAVM41, and AAVr3.45. Such AAV serotypes and derivatives/pseudotypes, and methods of producing such derivatives/pseudotypes are known in the art (see, e.g., Mol Ther. 2012 Apr;20(4):699-708. doi: 10.1038/mt.2011.287. Epub 2012 Jan 24. The AAV vector toolkit: poised at the clinical crossroads. Asokan A1, Schaffer DV, Samulski RJ.). In some embodiments, the rAAV particle is a pseudotyped rAAV particle, which comprises (a) a nucleic acid vector comprising ITRs from one serotype (e.g., AAV2) and (b) a capsid comprised of capsid proteins derived from another serotype (e.g., AAV1, AAV3, AAV4, AAV5, AAV6, AAV7, AAV8, AAV9, or AAV10). Methods for producing and using pseudotyped rAAV vectors are known in the art (see, e.g., Duan et al., J. Virol., 75:7662-7671, 2001; Halbert et al., J. Virol., 74:1524-1532, 2000; Zolotukhin et al., Methods, 28:158-167, 2002; and Auricchio et al., Hum. Molec. Genet., 10:3075-3081, 2001).

Exemplary rAAV nucleic acid vectors useful according to the disclosure include single-stranded (ss) or self-complementary (sc) AAV nucleic acid vectors, such as single-stranded or self-complementary recombinant viral genomes.

Methods of producing rAAV particles and nucleic acid vectors are also known in the art and commercially available (see, e.g., Zolotukhin et al. Production and purification of serotype 1, 2, and 5 recombinant adeno-associated viral vectors. Methods 28 (2002) 158-167; and U.S. Patent Publication Numbers US20070015238 and US20120322861, which are incorporated herein by reference; and plasmids and kits available from ATCC and Cell Biolabs, Inc.). For example, a plasmid containing the nucleic acid vector sequence may be combined with one or more helper plasmids, e.g., that contain a rep gene (e.g., encoding Rep78, Rep68, Rep52 and Rep40) and a cap gene (encoding VP1, VP2, and VP3, including a modified VP3 region as described herein), and transfected into a producer cell line such that the rAAV particle can be packaged and subsequently purified.

In some embodiments, the one or more helper plasmids includes a first helper plasmid comprising a rep gene and a cap gene and a second helper plasmid comprising a E1a gene, a E1b gene, a E4 gene, a E2a gene, and a VA gene. In some embodiments, the rep gene is a rep gene derived from AAV2 and the cap gene is derived from AAV2 and includes modifications to the gene in order to produce a modified capsid protein described herein. Helper plasmids, and methods of making such plasmids, are known in the art and commercially available (see, e.g., pDM, pDG, pDP1rs, pDP2rs, pDP3rs, pDP4rs, pDP5rs, pDP6rs, pDG(R484E/R585E), and pDP8.ape plasmids from PlasmidFactory, Bielefeld, Germany; other products and services available from Vector Biolabs, Philadelphia, PA; Cellbiolabs, San Diego, CA; Agilent Technologies, Santa Clara, Ca; and Addgene, Cambridge, MA; pxx6; Grimm et al. (1998), Novel Tools for Production and Purification of Recombinant Adenoassociated Virus Vectors, Human Gene Therapy, Vol. 9, 2745-2760; Kern, A. et al. (2003), Identification of a Heparin-Binding Motif on Adeno-Associated Virus Type 2 Capsids, Journal of Virology, Vol. 77, 11072-11081.; Grimm et al. (2003), Helper Virus-Free, Optically Controllable, and Two-Plasmid-Based Production of Adeno-associated Virus Vectors of Serotypes 1 to 6, Molecular Therapy,Vol. 7, 839-850; Kronenberg et al. (2005), A Conformational Change in the Adeno-Associated Virus Type 2 Capsid Leads to the Exposure of Hidden VP1 N Termini, Journal of Virology, Vol. 79, 5296-5303; and Moullier, P. and Snyder, R.O. (2008), International efforts for recombinant adeno-associated viral vector reference standards, Molecular Therapy, Vol. 16, 1185-1188).

An exemplary, non-limiting, rAAV particle production method is described next. One or more helper plasmids are produced or obtained, which comprise rep and cap ORFs for the desired AAV serotype and the adenoviral VA, E2A (DBP), and E4 genes under the transcriptional control of their native promoters. The cap ORF may also comprise one or more modifications to produce a modified capsid protein as described herein. HEK293 cells (available from ATCC®) are transfected via CaPO4-mediated transfection, lipids or polymeric molecules such as Polyethylenimine (PEI) with the helper plasmid(s) and a plasmid containing a nucleic acid vector described herein. The HEK293 cells are then incubated for at least 60 hours to allow for rAAV particle production. Alternatively, in another example Sf9-based producer stable cell lines are infected with a single recombinant baculovirus containing the nucleic acid vector. As a further alternative, in another example HEK293 or BHK cell lines are infected with a HSV containing the nucleic acid vector and optionally one or more helper HSVs containing rep and cap ORFs as described herein and the adenoviral VA, E2A (DBP), and E4 genes under the transcriptional control of their native promoters. The HEK293, BHK, or Sf9 cells are then incubated for at least 60 hours to allow for rAAV particle production. The rAAV particles can then be purified using any method known the art or described herein, e.g., by iodixanol step gradient, CsCl gradient, chromatography, or polyethylene glycol (PEG) precipitation.

As used herein, the terms "engineered" and "recombinant" cells are intended to refer to a cell into which an exogenous polynucleotide segment (such as DNA segment that leads to the transcription of a biologically active molecule) has been introduced. Therefore, engineered cells are distinguishable from naturally occurring cells, which do not contain a recombinantly introduced exogenous DNA segment. Engineered cells are, therefore, cells that comprise at least one or more heterologous polynucleotide segments introduced through the hand of man.

To express a therapeutic agent in accordance with the present invention one may prepare a tyrosine capsid-modified rAAV particle containing an expression vector that comprises a therapeutic agent-encoding nucleic acid segment under the control of one or more promoters. To bring a sequence "under the control of' a promoter, one positions the 5' end of the transcription initiation site of the transcriptional reading frame generally between about 1 and about 50 nucleotides "downstream" of (*i.e.,* 3' of) the chosen promoter. The "upstream" promoter stimulates transcription of the DNA and promotes expression of the encoded polypeptide. This is the meaning of "recombinant expression" in this context. Particularly preferred recombinant nucleic acid vector constructs are those that comprise an rAAV nucleic acid vector that contains a therapeutic gene of interest operably linked to one or more promoters that is capable of expressing the gene in one or more selected mammalian cells. Such nucleic acid vectors are described in detail herein.

The genetic constructs of the present invention may be prepared in a variety of compositions, and may also be formulated in appropriate pharmaceutical vehicles for administration to human or animal subjects. The rAAV molecules of the present invention and compositions comprising them provide new and useful therapeutics for the treatment, control, and amelioration of symptoms of a variety of disorders, diseases, injury, and/or dysfunctions of the mammalian nervous system, and in particular, in the treatment or amelioration of MS.

In some embodiments, the number of rAAV particles administered to a subject may be on the order ranging from 10⁶ to 10¹⁴ particles/ml or 10³ to 10¹⁵ particles/ml, or any values therebetween for either range, such as for example, about 10⁶, 10⁷, 10⁸, 10⁹, 10¹⁰, 10¹¹, 10¹², 10¹³, or 10¹⁴ particles/ml. In one embodiment, rAAV particles of higher than 10¹³ particles/ml may be administered. In some embodiments, the number of rAAV particles administered to a subject may be on the order ranging from 10⁶ to 10¹⁴ vector genomes(vgs)/ml or 10³ to 10¹⁵ vgs/ml, or any values therebetween, such as for example, about 10⁶, 10⁷, 10⁸, 10⁹, 10¹⁰, 10¹¹, 10¹², 10¹³, or 10¹⁴ vgs/ml. In one embodiment, rAAV particles of higher than 10¹³ vgs/ml are administered. The rAAV particles can be administered as a single dose, or divided into two or more administrations as may be required to achieve therapy of the particular disease or disorder being treated. In some embodiments, 0.0001 ml to 10 mls, e.g., 0.001ml, 0.01ml, 0.1ml, 1 ml, 2ml, 5ml or 10 ml, are delivered to a subject. In some embodiments, the number of rAAV particles administered to a subject may be on the order ranging from 10⁶-10¹⁴ vg/kg, or any values therebetween, such as for example, about 10⁶, 10⁷, 10⁸, 10⁹, 10¹⁰, 10¹¹, 10¹², 10¹³, or 10¹⁴ vgs/mg.

In some embodiments, the disclosure provides formulations of one or more viral-based compositions disclosed herein in pharmaceutically acceptable solutions for administration to a cell or an animal, either alone or in combination with one or more other modalities of therapy, and in particular, for therapy of human cells, tissues, and diseases affecting man.

If desired, rAAV particles described herein may be administered in combination with other agents as well, such as, e.g., proteins or polypeptides or various pharmaceutically-active agents, including one or more systemic or topical administrations of therapeutic polypeptides, biologically active fragments, or variants thereof. In fact, there is virtually no limit to other components that may also be included, given that the additional agents do not cause a significant adverse effect upon contact with the target cells or host tissues. The rAAV particles may thus be delivered along with various other agents as required in the particular instance. Such compositions may be purified from host cells or other biological sources, or alternatively may be chemically synthesized as described herein.

Formulation of pharmaceutically-acceptable excipients and carrier solutions is well-known to those of skill in the art, as is the development of suitable dosing and treatment regimens for using the particular compositions described herein in a variety of treatment regimens, including e.g., oral, parenteral, intravitreal, intraocular, intravenous, intranasal, intra-articular, and intramuscular administration and formulation.

Typically, these formulations may contain at least about 0.1% of the therapeutic agent (e.g., rAAV particle) or more, although the percentage of the active ingredient(s) may, of course, be varied and may conveniently be between about 1 or 2% and about 70% or 80% or more of the weight or volume of the total formulation. Naturally, the amount of therapeutic agent(s) in each therapeutically-useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

In certain circumstances it will be desirable to deliver rAAV particles in suitably formulated pharmaceutical compositions disclosed herein either subcutaneously, intraocularly, intravitreally, parenterally, subcutaneously, intravenously, intracerebro-ventricularly, intramuscularly, intrathecally, orally, intraperitoneally, by oral or nasal inhalation, or by direct injection to one or more cells, tissues, or organs by direct injection. The pharmaceutical forms of the compositions suitable for injectable use include sterile aqueous solutions or dispersions. In some embodiments, the form is sterile and fluid to the extent that easy syringability exists. In some embodiments, the form is stable under the conditions of manufacture and storage and is preserved against the contaminating action of microorganisms, such as bacteria and fungi. The carrier can be a solvent or dispersion medium containing, for example, water, saline, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and by the use of surfactants.

The term "carrier" refers to a diluent, adjuvant, excipient, or vehicle with which the rAAV particle is administered. Such pharmaceutical carriers can be sterile liquids, such as water and oils, including those of petroleum oil such as mineral oil, vegetable oil such as peanut oil, soybean oil, and sesame oil, animal oil, or oil of synthetic origin. Saline solutions and aqueous dextrose and glycerol solutions can also be employed as liquid carriers. Other exemplary carriers include phosphate buffered saline, HEPES-buffered saline, and water for injection, any of which may be optionally combined with one or more of calcium chloride dihydrate, disodium phosphate anhydrous, magnesium chloride hexahydrate, potassium chloride, potassium dihydrogen phosphate, sodium chloride, or sucrose.

The compositions of the present disclosure can be administered to the subject being treated by standard routes including, but not limited to, pulmonary, intranasal, oral, inhalation, parenteral such as intravenous, topical, transdermal, intradermal, transmucosal, intraperitoneal, intramuscular, intracapsular, intraorbital, intravitreal, intracardiac, transtracheal, subcutaneous, subcuticular, intraarticular, subcapsular, subarachnoid, intraspinal, epidural and intrasternal injection. In some embodiments, the composition is administered intravenously, by hepatic artery infusion, portal vein injection, or intrasplenic injection. In some embodiments, the composition comprises a AAV8 rAAV particle comprising a rAAV nucleic acid vector as described herein, and the composition is administered intravenously.

For administration of an injectable aqueous solution, for example, the solution may be suitably buffered, if necessary, and the liquid diluent first rendered isotonic with sufficient saline or glucose. These particular aqueous solutions are especially suitable for intravenous, intramuscular, intravitreal, subcutaneous and intraperitoneal administration. In this connection, a sterile aqueous medium that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage may occur depending on the condition of the subject being treated. The person responsible for administration will, in any event, determine the appropriate dose for the individual subject. Moreover, for human administration, preparations should meet sterility, pyrogenicity, and the general safety and purity standards as required by, e.g., FDA Office of Biologics standards.

Sterile injectable solutions may be prepared by incorporating the rAAV particles in the required amount in the appropriate solvent with several of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, exemplary methods of preparation are vacuum-drying and freeze-drying techniques which yield a powder of the active ingredient plus any additional desired ingredient from a previously sterile-filtered solution thereof.

The amount of rAAV particle compositions and time of administration of such compositions will be within the purview of the skilled artisan having benefit of the present teachings. It is likely, however, that the administration of therapeutically-effective amounts of the disclosed compositions may be achieved by a single administration, such as for example, a single injection of sufficient numbers of viral particles to provide therapeutic benefit to the patient undergoing such treatment. Alternatively, in some circumstances, it may be desirable to provide multiple, or successive administrations of the compositions, either over a relatively short, or a relatively prolonged period of time, as may be determined by the medical practitioner overseeing the administration of such compositions.

The composition may include rAAV particles or nucleic acid vectors either alone, or in combination with one or more additional active ingredients, which may be obtained from natural or recombinant sources or chemically synthesized.

In accordance with the present invention, polynucleotides, nucleic acid segments, nucleic acid sequences, and the like, include, but are not limited to, DNAs (including and not limited to genomic or extragenomic DNAs), genes, peptide nucleic acids (PNAs), RNAs (including, but not limited to, rRNAs, mRNAs and tRNAs), nucleosides, and suitable nucleic acid segments either obtained from natural sources, chemically synthesized, modified, or otherwise prepared or synthesized in whole or in part by the hand of man.

Unless defined otherwise, all technical and scientific terms used herein have the same meaning as commonly understood by one of ordinary skill in the art to which this invention belongs. Although any methods and compositions similar or equivalent to those described herein can be used in the practice or testing of the present invention, the preferred methods and compositions are described herein. For purposes of the present invention, the following terms are defined below:

The term "subject," as used herein, describes an organism, including mammals such as primates, to which treatment with the compositions according to the present invention can be provided. Mammalian species that can benefit from the disclosed methods of treatment include, but are not limited to, humans; apes; chimpanzees; orangutans; monkeys; domesticated animals such as dogs and cats; livestock such as horses, cattle, pigs, sheep, goats, and chickens; and other animals such as mice, rats, guinea pigs, and hamsters. In some embodiments, the subject has, is suspected of having, is at risk for developing, or has been diagnosed with an autoimmune disease or disorder, such as multiple sclerosis, disseminated sclerosis, or encephalomyelitis disseminata. In some embodiments, the subject has an autoimmune disease or disorder, such as multiple sclerosis, disseminated sclerosis, or encephalomyelitis disseminata. Other exemplary autoimmune diseases include type 1 diabetes, Grave's disease, arthritis (e.g., rheumatoid arthritis or PGIA), autoimmune uveitis, Peripheral Neuropathy, Myasthenia gravis, Lupus, and Crohn's disease. In some embodiments, an autoimmune disease or disorder is associated with an infection (e.g., a microbial or viral infection).

The term "treatment" or any grammatical variation thereof (*e.g.,* treat, treating, and treatment *etc.*), as used herein, includes but is not limited to, alleviating a symptom of a disease or condition; and/or reducing, suppressing, inhibiting, lessening, ameliorating or affecting the progression, severity, and/or scope of a disease or condition.

The term "effective amount," as used herein, refers to an amount that is capable of treating or ameliorating a disease or condition or otherwise capable of producing an intended therapeutic effect.

The term "promoter," as used herein refers to a region or regions of a nucleic acid sequence that regulates transcription.

The term "regulatory element," as used herein, refers to a region or regions of a nucleic acid sequence that regulates transcription. Exemplary regulatory elements include, but are not limited to, enhancers, post-transcriptional elements, transcriptional control sequences, and such like.

The tern "vector," as used herein, refers to a nucleic acid molecule (typically comprised of DNA) capable of replication in a host cell and/or to which another nucleic acid segment can be operatively linked so as to bring about replication of the attached segment. A plasmid, cosmid, or a virus is an exemplary vector.

The term "substantially corresponds to," "substantially homologous," or "substantial identity," as used herein, denote a characteristic of a nucleic acid or an amino acid sequence, wherein a selected nucleic acid or amino acid sequence has at least about 70 or about 75 percent sequence identity as compared to a selected reference nucleic acid or amino acid sequence. More typically, the selected sequence and the reference sequence will have at least about 76, 77, 78, 79, 80, 81, 82, 83, 84 or even 85 percent sequence identity, and more preferably, at least about 86, 87, 88, 89, 90, 91, 92, 93, 94, or 95 percent sequence identity. More preferably still, highly homologous sequences often share greater than at least about 96, 97, 98, or 99 percent sequence identity between the selected sequence and the reference sequence to which it was compared.

The percentage of sequence identity may be calculated over the entire length of the sequences to be compared, or may be calculated by excluding small deletions or additions which total less than about 25 percent or so of the chosen reference sequence. The reference sequence may be a subset of a larger sequence, such as a portion of a gene or flanking sequence, or a repetitive portion of a chromosome. However, in the case of sequence homology of two or more polynucleotide sequences, the reference sequence will typically comprise at least about 18-25 nucleotides, more typically at least about 26 to 35 nucleotides, and even more typically at least about 40, 50, 60, 70, 80, 90, or even 100 or so nucleotides.

When highly-homologous fragments are desired, the extent of percent identity between the two sequences will be at least about 80%, preferably at least about 85%, and more preferably about 90% or 95% or higher, as readily determined by one or more of the sequence comparison algorithms well-known to those of skill in the art, such as *e.g.,* the FASTA program analysis described by Pearson and Lipman (1988).

The term "operably linked," as used herein, refers to that the nucleic acid sequences being linked are typically contiguous, or substantially contiguous, and, where necessary to join two protein coding regions, contiguous and in reading frame. However, since enhancers generally function when separated from the promoter by several kilobases and intronic sequences may be of variable lengths, some polynucleotide elements may be operably linked but not contiguous.

The term "biologically active" as used herein, refers to a variant nucleic acid or protein sequence that has substantially the same activity, such as reduction of clinical severity of EAE in a mouse model or induction of T regulatory cells as described in Example 1, Example 2, Example 3, or Example 4 below, as a nucleic acid or protein as described herein (e.g., has substantially the same or the same activity as a MOG, PLP, or MBP nucleic acid or protein described herein).

### EXAMPLES

The following examples are included to demonstrate preferred embodiments of the invention. It should be appreciated by those of skill in the art that the techniques disclosed in the examples that follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLE 1 - RE-ESTABLISHING IMMUNE TOLERANCE TO NEUROANTIGENS BY AAV GENE THERAPY

The inventors have demonstrated that hepatocyte-restricted expression of an AAV-delivered neuroantigen establishes persistent immunological tolerance mediated by antigen-specific Tregs capable of preventing and reversing EAE in mice. This example describes the development of a protocol that persistently induces Tregs *in vivo* and prevents disease development in a murine model of MS. The example also determines if tolerance can induce remission of pre-existing EAE disease and substantially reduce clinical and tissue-associated pathology.

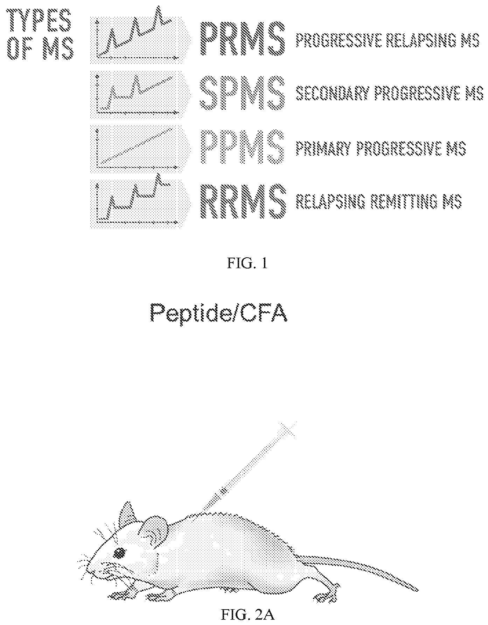

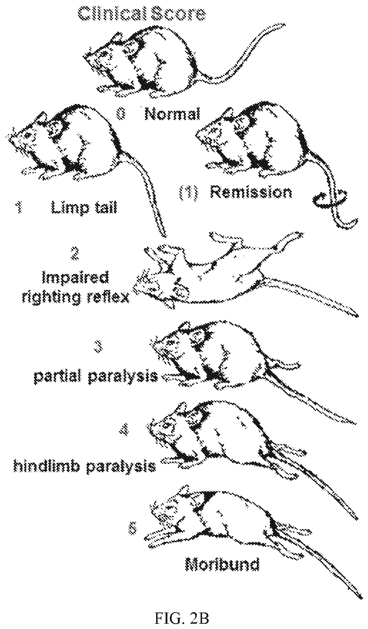

Neurodegenerative disease such as Multiple sclerosis (MS) is characterized by chronic infiltration of the CNS by pathogenic autoreactive lymphocytes that recognize neuroantigens. Functional defects in the endogenous regulatory T cells (Tregs) leading to a failure of central and/or peripheral mechanisms required for maintaining immunological tolerance combined with T cells recognizing myelin protein peptides are implicated in the pathogenesis of the disease. In C57BL/6 mice, experimental autoimmune encephalomyelitis (EAE) induced by myelin oligodendrocyte glycoprotein (MOG) produces a CD4 T cell-mediated inflammatory CNS disease that serves as a relevant model for MS (FIG. 1 and FIGs. 2A and 2B).

Hepatic gene transfer with AAV vectors containing liver specific promoters can produce stable transgene expression and induce a robust antigen-specific immune tolerance to a variety of therapeutic proteins. It has been reported that induced Tregs not only suppress cellular immune responses against the transgene product but can also suppress humoral responses. Importantly, it has been shown that immune tolerance established by antigen expression in the liver is maintained even when the antigen was subsequently expressed in a highly immunogenic manner in other organs, such as skeletal muscle or intravenously.

The development of protocols that stimulate an increase in Treg numbers and/or their function has become a focus in treating autoimmune disease. Many of the beneficial effects of currently approved immune-modulators used in the treatment of MS are associated with restoring Treg homeostasis. This example demonstrates that liver directed AAV gene therapy represents a novel approach to halt disease progression by restoring normal Treg function at disease onset.

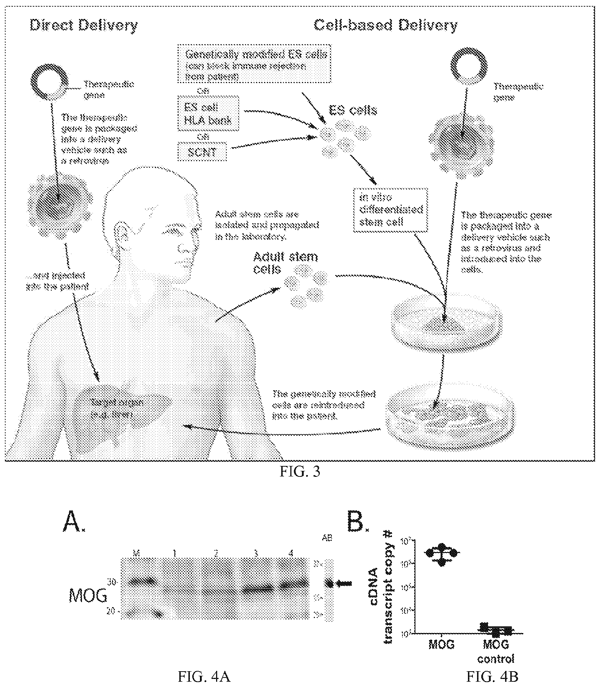

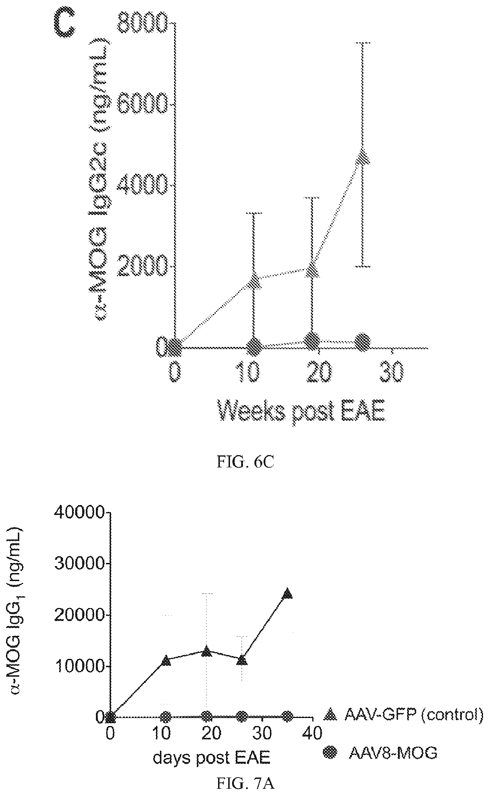

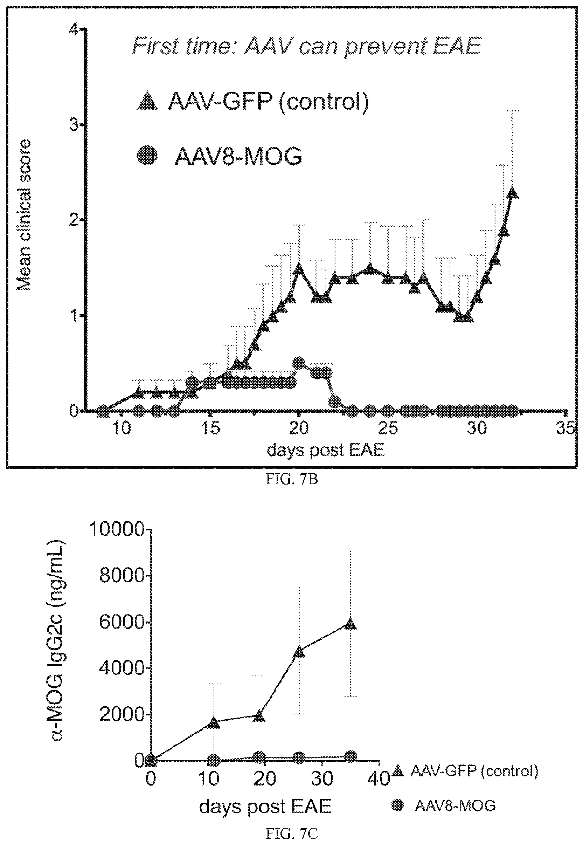

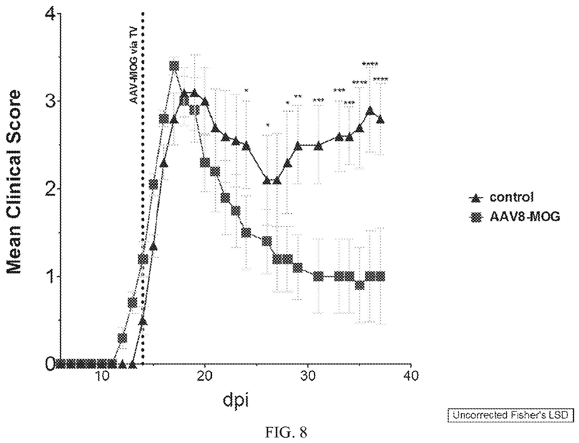

First, an AAV8-MOG vector was generated, and hepatic expression of the transgene in mice was validated by western blot and qPCR analysis (FIG. 4). Next, to determine if hepatic expression of MOG can provide protection against the development of EAE mice were injected with either AAV8-MOG or -GFP vector. 2 weeks later EAE was induced and the mice were monitored and scored according to the classic scale for clinical signs of EAE. Plasma was obtained at 0, 7 and 14 days post EAE or at 0, 11, 19, 26, and 35 days post EAE. The results revealed that mice receiving AAV8-MOG were clearly protected from developing EAE. Furthermore, these mice also did not produce any anti-MOG IgG1 or IgG2c autoantibodies. In contrast, those mice receiving the control vector developed severe EAE with elevated antibody titers (FIGs. 7 and 8).

### EXAMPLE 2 -THERAPEUTIC MOLECULES FOR AAV-BASED GENE THERAPY OF MS

This example shows that liver directed gene transfer using an AAV vector expressing a neuro-antigen is capable of suppressing inflammation in the CNS and preventing EAE. Importantly, using AAV to express a full-length neuro-protein will enable greater applicability across MS-associated HLA haplotypes. Ongoing plans are to evaluate reversal of pre-existing EAE and functional analysis of the interplay of effector (Th1/Th17) cells and Tregs.

Using sequences for full length proteins, avoiding HLA/MHC restrictions
**MBP sequence in vector:** 
MGNHSGKRELSAEKASKDGEIHRGEAGKKRSVGKLSQTASEDSDVFGEADAIQNNGTSAEDT
AVTDSKHTADPKNNWQGAHPADPGNRPHLIRLFSRDAPGREDNTFKDRPSESDELQTIQEDP
TAASGGLDVMASQKRPSQRSKYLATASTMDHARHGFLPRHRDTGILDSIGRFFSGDRGAPKR
GSGKVSSEP* (SEQ ID NO:1)
**PLP sequence in vector:** 
MGLLECCARCLVGAPFASLVATGLCFFGVALFCGCGHEALTGTEKLIETYFSKNYQDYEYLI
NVIHAFQYVIYGTASFFFLYGALLLAEGFYTTGAVRQIFGDYKTTICGKGLSATVTGGQKGR
GSRGQHQAHSLERVCHCLGKWLGHPDKFVGITYALTVVWLLVFACSAVPVYIYFNTWTTCQS
IAFPSKTSASIGSLCADARMYGVLPWNAFPGKVCGSNLLSICKTAEFQMTFHLFIAAFVGAA
(SEQ ID NO:2)
**MOG sequence in vector:** 
MACLWSFSLPSCFLSLLLLLLLQLSCSYAGQFRVIGPGYPIRALVGDEAELPCRISPGKN
ATGMEVGWYRSPFSRVVHLYRNGKDQDAEQAPEYRGRTELLKETISEGKVTLRIQNVRFS
DEGGYTCFFRDHSYQEEAAMELKVEDPFYWVNPGVLTLIALVPTILLQVSVGLVFLFLQH
RLRGKLRAEVENLHRTFDPHFLRVPCWKITLFVIVPVLGPLVALIICYNWLHRRLAGQFL
EELRNPL (SEQ ID NO:3)
EAE inducing peptide in SJL mice PLP 139-151 HCLGKWLGHPDKF (SEQ ID NO:4)
EAE inducing peptide in C57BL mice NTWTCQSIAFP (SEQ ID NO:5) or PLP₁₇₈₋₁₉₁ NTWTTCQSIAFPSK (SEQ ID NO: 37)
C57BL: MOG35-55 MEVGWYRSPFSRVVHLYRNGK (SEQ ID NO:6) SJL: MOG92-106 DEGGYTCFFRDHSYQ (SEQ ID NO:7)

### EXAMPLE 3 -RAAV8 VECTORS FOR GENE THERAPY OF MS

AAV8 vectors can stably express a neuro-protein in hepatocytes. AAV8-MOG can prevent the development of EAE, and AAV8-MOG can abrogate clinical symptoms of established EAE.

This example describes the development of a (pre)clinically relevant therapy using viral gene transfer that will result in the induction and expansion of antigen-specific T cells, re-establishing immunological tolerance as a treatment for multiple sclerosis. The approach has broad application as it uses full length myelin oligodendrocyte glycoprotein (MOG) protein and thus abrogates the needs for identifying HLA/MHC specific epitopes for inducing antigen specific Tregs. The knowledge gained from the work presented here will have the potential of creating a new line of treatment protocols for patients with MS as well as advance the research for both the MS and gene therapy fields.

Collectively, there is clear rationale for therapeutic approaches that are multifactorial. The present invention provides a novel therapy that not only focuses on reducing CD4⁺ T cells, but that can also target the effect of CD8⁺ T cells, B cells, and B cell derived components of the immune system. Previous data, suggests that the presentation of neuro-antigens within liver delivered by AAV should have significant clinical relevance as a therapeutic intervention for MS and other autoimmune diseases. This application of gene therapy represents a new and innovative treatment option for MS.

### EXPERIMENTAL METHODS

***Prevention of EAE:*** C57B1/6 mice will be injected with AAV8-MOG or control vector for hepatocyte-specific expression. At 4 weeks after gene transfer, induction of EAE will begin. Mice will be monitored daily and neurological impairment will be recorded on a detailed clinical scale. Weekly, blood/serum will be collected and analyzed for frequency of activated CD4, CD8, and Tregs by flow cytometric analysis, and α-MOG antibody formation via ELISA. Upon termination, histology of harvested tissues will be evaluated for transgene expression (liver), magnitude and phenotype of infiltration of inflammatory cells, demyelination, and white matter damage (CNS). Establishing a baseline correlation of histology to clinical score (especially in control mice) will be essential for identifying success in subsequent aims.

***Reversal of EAE:*** In this study, induction of EAE in mice is first performed. Upon the first signs of EAE, mice will be randomly selected to receive hepatic gene transfer using AAV8-MOG or control vector. A detailed clinical assessment will be recorded daily. At various time points, blood/serum will be collected and analyzed as above. Upon sacrificing, liver and CNS tissue will be harvested and preserved for pathological and histochemical analysis.

***Ex vivo functional analysis of tolerogenic Tregs:*** To determine if the AAV8-MOG induced Tg-specific Tregs are immunosuppressive, GFP⁺Tregs isolated by FACS from transgenic mice ("Foxp3^{EGFP}" B6.Cg-*Foxp3^{tm2Tch}*/J) that received vector 4 wks earlier will be co-cultured with allogeneic splenocytes obtained from 2D2-TCR mice (MOG specific TCR) in the presence of MOG peptide. Cells and culture supernatant will be analyzed for activation, apoptosis, Th1/Th2/Th17 cytokines, or CTL activity via specific assays.

***In vivo adoptive transfer of Tregs:*** To test whether the immunosuppressive function of Tg-specific Tregs are able to attenuate disease progression, GFP⁺Tregs isolated as above, will be adoptively transferred into (a). naive mice that will subjected to EAE induction 24hrs later AND (b). mice that have undergone MOG induced EAE. These conditions will mirror the events from Aim 1.1 prevention and 1.2 reversal. At various time points, blood/serum will be collected; and liver and CNS tissue will be harvested and analyzed as above.

The overall theme of the present invention is the development of a gene therapy based method for *in vivo* induction of endogenous antigen (Ag)-specific regulatory T-cells (Tregs) using liver-directed Adeno-associated virus (AAV) gene therapy, as a novel treatment strategy for autoimmune diseases, e.g., multiple sclerosis (MS).

As noted above, MS is an autoimmune neurodegenerative disease of the central nervous system (CNS) in which the etiology is not well understood. Although, auto-aggressive CD4⁺ T cells play a central role, the breakdown of immune tolerance mechanisms that permits activation of naive myelin-specific T cells is considered an initial step in the pathogenesis of MS. A number of pivotal studies in rodent models have substantiated that Ag-specific Tregs have a significant role in modulating autoimmune CNS disease and can be highly effective at treating MS.¹⁻⁵ Consequentially, there has been a major focus in developing protocols that stimulate Treg numbers and their function. Unfortunately, successful therapeutic use of Tregs has been limited by the lack of safe and effective Ag-specific protocols for isolation and expansion that are suitable for translation.

Using the AAV gene transfer platform, it has been clearly demonstrated that hepatocyte-restricted transgene expression from an optimized AAV vector can reliably induce immune tolerance to various therapeutic proteins, including coagulation factor IX (F.IX), α-1-antitrypsin, erythropoietin, and lysosomal storage enzymes, among others.⁶ Tolerance induction after hepatic gene transfer involves a combination of mechanisms. Importantly, AAV induced tolerance is mediated by Ag-specific CD4⁺CD25⁺FoxP3⁺ Tregs, which is critically dependent on achieving and maintaining adequate hepatocyte-restricted transgene expression.⁷⁻⁹ It has also been demonstrated that AAV induced Tregs can actively suppress antibody formation and cytotoxic CD8⁺ T cell responses against the transgene product.^{7, 10, 11} Tolerized animals fail to form antibodies to the transgene even after subsequent attempts to immunize with protein formulated in adjuvant.¹⁰⁻¹² Efficient hepatic gene transfer induces a TGF-β dependent CD4⁺CD25⁺FoxP3⁺ Treg response that confers a dominant state of Ag-specific immune tolerance that is maintained even when the antigen was later introduced in other tissues in a highly immunogenic manner.^{7, 12} Induction of programmed cell death of effector T cells further tilts the balance toward tolerance, which is effectively enforced by induced Treg.^{13, 14} Published data have demonstrated that hepatic AAV tolerance can also reverse pre-existing immune responses to F.IX in a hemophilia B mouse model.¹⁵ These now well-established concepts have been further supported by results from other laboratories and led to the development of several immune tolerance protocols for genetic diseases.¹⁶⁻³⁷

Over 400,000 people in the United States currently are living with MS, and 10,000 new cases are diagnosed each year. With a 1:600-800 lifetime risk of developing the disease, MS is the most common cause of neurologic disability in young adults between 18 and 45 years of age. This demographic represents the majority of the adult workforce in the United States; therefore, the direct and indirect costs of health care for this population currently are estimated at $12 billion annually.³⁸

***Multiple sclerosis (MS) is a protracted, immune-mediated disease of the CNS.*** MS is a neuroinflammatory autoimmune disease in which T cell driven inflammation leads to demyelination and damage of axons. Although the exact pathogenesis of MS remains unknown, it is believed that myelin-specific CD4⁺ T cells play a central role in initiating and orchestrating CNS inflammation. A failure of central and peripheral mechanisms (particularly Tregs) to maintain self-tolerance and control potentially pathogenic auto-reactive lymphocytes is thought to be a key event in the development and pathogenesis of MS.^{4, 39-41} Several studies using *in vitro* suppression assays have documented functional impairments of Tregs from MS patients.^{42, 43} Experiments in mice using adoptive transfer of myelin-specific Tregs or Treg depletion have also provided evidence that Tregs can control the development and severity of experimental autoimmune encephalomyelitis (EAE) and accumulate within the CNS during the recovery.⁴⁴ It has also been shown that transgenic mice expressing myelin basic protein (MBP) could prevent the onset of EAE disease in mice in a Treg dependent process.^{45, 46} In fact, the mechanism-of-action for several of the currently approved immune-modulators used in the treatment of MS are associated with restoring Treg homeostasis.^{39, 47,} 48

Cumulatively, the literature clearly supports the concept that Treg cells influence the susceptibility and progression of disease. Recent advances have led to the recognition that Ag-specific Tregs represents an ideal form of cell therapy for MS. However, Tregs are still among the least understood T cell subsets, and consequently the most difficult to use for therapeutic applications.

***Gene therapy with AAV vectors induces antigen-specific immune tolerance.*** Gene therapy continues to be a proven and powerful new tool for the treatment of a broad spectrum of diseases.⁴⁹ AAV vectors specifically have had great successes with *in vivo* gene transfer to a variety of target tissues.¹² For example, AAV gene transfer to retinal epithelial cells restores vision in children with Leber's Congenital Amaurosis (LCA) and with Choroideremia.^{50, 51} An AAV vector for treatment of lipoprotein lipase is the first gene therapy drug approved in the Western world ("Glybera").⁵² Gene therapy by hepatic AAV administration has resulted in sustained expression of factor IX (F.IX) at levels of >5% of normal in hemophilia B patients, changing their bleeding phenotype from severe to mild.⁴⁸ Hepatic AAV gene transfer promotes tolerance via induction of transgene product-specific Treg, a phenomenon that can be exploited for the treatment of MS.^{6, 21, 34, 35, 49, 53}

Effective therapy for established EAE needs to consider induction of multiple direct and indirect (cross-tolerance) regulatory mechanisms, including the induction of antigen (Ag)-specific CD4⁺CD25⁺FoxP3⁺ Tregs across multiple endogenous myelin epitopes (epitope spreading). The literature supports the idea that Tregs are potent suppressors of EAE, and essential to establish disease remission. However, very few studies have addressed how to generate such Ag-specific Tregs that is both reliable and translatable. Based on previous work, hepatic gene transfer using AAV8 vectors to express full-length myelin-associated proteins will induce Ag-specific Tregs across multiple endogenous epitopes in a manner that has been shown to be safe, feasible, and long lasting. This study is innovative in several respects. (i) This is the first time a clinically proven AAV vector technique is used to re-establish immunological tolerance in the context of an autoimmune disease. (ii) exemplary AAV8 vectors have been designed to express a full-length neuro-protein (myelin oligodendrocyte glycoprotein (MOG) or proteolipid proteins (PLP)) thus abrogating the need for identifying HLA/MHC specific epitopes and enhancing the potential for success. (iii) Based on published data, incorporating transient immune modulation using the FDA approved mTOR inhibitor, rapamycin, should provide a synergistic effect, facilitating tolerance induction to neuroantigens by further tipping the balance from Teff to Treg *in vivo.*⁵⁴⁻⁵⁶

The inventors contemplate that AAV8 liver gene transfer of a neural protein (PLP and MOG) induces activation of Ag-specific Tregs, sufficient to re-establish immune tolerance and abrogate disease progression in the CNS of a murine model for MS.

The basis for this proposal is founded on precedent, and recently-published studies in which relevant findings are presented in the following:

Hepatic gene transfer with AAV vectors can reliably induce a robust antigen-specific immune tolerance in experimental animals to a variety of therapeutic proteins.^{7, 9, 13, 54, 57} Here, tolerance was characterized by lack of antibody formation, helper T cell response, or CTL response to the transgene product, even after subsequent challenge with protein in adjuvant. Using mice transgenic for a T cell receptor, evidence of anergy and deletion of transgene product-specific CD4 T cells was found.

That immune tolerance established by hepatic transgene expression is maintained even when the antigen was subsequently expressed in a highly immunogenic manner in other organs, such as skeletal muscle, or even delivered intravenously.⁷ These results revealed that liver directed gene therapy could abrogate potential cytotoxic CD8 T cell responses, indicating that the range of immune tolerance extends beyond the level of antigen expression initially achieved by hepatic gene transfer.

Hepatic AAV gene transfer efficiently and rapidly reversed pre-existing high antibodies titers and provided long-term correction of haemostasis in a murine hemophilia B model.^{15, 56} High levels of transgene protein suppressed memory B cells and increased Treg induction, indicating direct and indirect mechanisms of suppression of inhibitor formation. This finding is quite significant since there is an increasing body of evidence that B cells and autoantibodies may play a pathogenic role in demyelinating disease.^{58, 59}

***Immune tolerance induction by hepatic AA Vgene transfer does not require** protein **to be secreted.*** Although hepatic expression is crucial for tolerance induction, secretion from hepatocytes for systemic delivery of the transgene product is not required. Expression of a cytoplasmic a neo-antigen in as few as 3% of the hepatocytes is sufficient to induce Tregs and provide long-term suppression of inflammatory responses.⁵⁷

### RESULTS

***Successfully establishing multiple models of EAE induction:*** EAE is a widely accepted experimental mouse model of multiple sclerosis that is induced in susceptible animals by immunization with central nervous system antigens. EAE is an autoimmune disease that is mediated by CD4⁺ T helper 1 (T_{H}1) cells and interleukin-17 producing T_{H}17 cells that are reactive to components of the myelin sheath. The cells infiltrate the nervous parenchyma, release pro-inflammatory cytokines and chemokines, promote leukocyte infiltration and contribute to demyelination.

EAE can be induced in various strains of mice using different neuro-proteins emulsified in complete Freud's adjuvant (CFA). Disease progression and pathology manifests differently with each combination. For example, EAE induced by (i) MOG produces encephalitogenic T-cells and demyelinating autoantibodies in C57BL/6 mice. The resulting disease is a chronic-progressive disease characterized by axonal demyelination and white matter lesions in the spinal cord and is generally considered to be a relevant model for human immune-mediated demyelinating disease.⁶⁰ EAE can also be induced in SJL (H-2s) mice using the major encephalitogenic *(ii)* PLP peptide (PLP₁₃₉₋₁₅₁). Here the disease is characterized by a relapsing-remitting course of paralysis, which allows assessment of the efficacy of various immune regulatory strategies in a re-occurring disease setting.

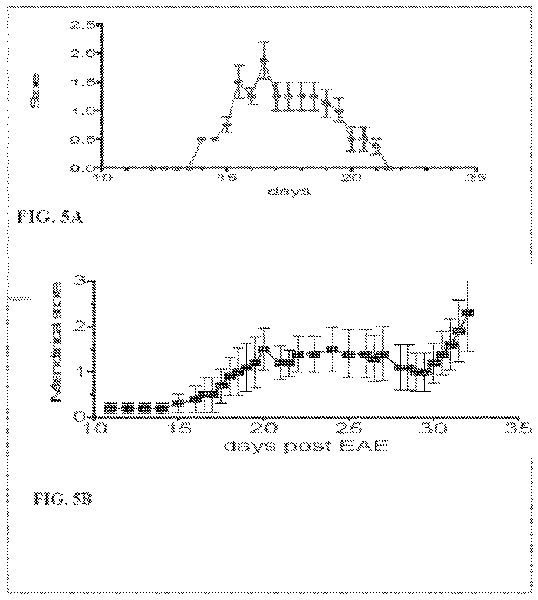

In these studies, the inventors demonstrated the timeline and clinical scoring for successful induction of EAE disease in two different mouse strains. In one experiment, 8-week-old female mice were injected subcutaneously with 200µg myelin peptide emulsified in CFA containing 4mg/ml Mycobacterium tuberculosis. Clinical signs of EAE began 12 days later at which time mice were evaluated twice daily. Mice were scored according to the severity of the clinical signs (FIG. 5A). In a similar experiment, EAE was induced in C57BL/6 mice (n = 5) using MOG in order to develop a chronic progressive EAE disease (FIG. 5B)

***Novel AAV8 vectors transduce mouse hepatocytes efficiently and express the delivered neural protein:***_AAV is a non-pathogenic single stranded DNA parvovirus with a genome size of approximately 4.7kb. Serotypes with distinct tissue tropisms have been isolated from multiple vertebrate species, including humans. Viral vectors derived from AAV are devoid of viral genes and instead contain an expression cassette for the gene of interest, which is limited to ∼5kb in length. In these studies, an AAV8 serotype vector was chosen because it has strong natural tropism for hepatocytes after peripheral vein administration, avoiding the need for an invasive procedure. Additionally, it fails to transduce professional antigen presenting cells (APCs). The engineered vector constructs include a strong and highly hepatocyte-specific promoter.¹⁰

The newly synthesized vectors were evaluated for transduction efficiency. To demonstrate efficacy, the inventors assessed whether mouse hepatocytes could be transduced and express the neuro-protein transgene following tail vein injection. A group of mice was injected with 1 × 10¹¹ vector particles of AAV8-ApoE/hAAT-MOG. Two weeks later, using liver lysates evidence of hepatic expression of MOG was probed by both western blot and qPCR analysis. The results clearly demonstrate the ability this novel vector to stably produce hepatic expression of the neuro-antigen after liver gene transfer (FIG. 4A and FIG. 4B).

***AAV8-MOG produces hepatic transgene expression that can prevent the establishment of EAE:*** Previously others have shown that ectopic expression of a myelin-associated protein using various transient methodologies promoted resistance to EAE.^{18, 28, 45, 61} Unfortunately, these prior approaches have not developed into practical therapies for human autoimmune disease. Prior to this invention, the ability of AAV liver gene transfer to induce antigen specific suppression of autoimmune disease went untested in the scientific community.

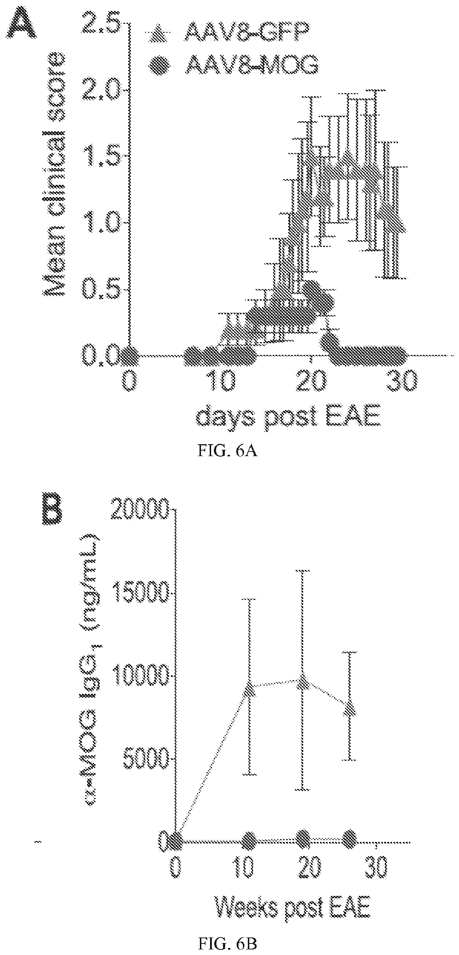

To further support this invention, a pilot study was performed. A small number of mice (n=5) were intravenously injected with 10¹¹ vector particles *via* the tail vein with either AAV8-MOG or AAV8-GFP (control) vector. Two weeks later, EAE was induced using MOG in CFA as previously performed. Plasma samples were obtained at 0, 7 and 14 days post EAE induction or at 0, 11, 19, 26, and 35 days post EAE induction. The mice that received AAV8-MOG were essentially protected from developing EAE (FIG. 6A, FIG. 6B, and FIG. 6C). In contrast, those mice receiving the control vector developed severe EAE with elevated antibody titers. This data indicates that the vectors described herein not only express in the liver, but also had an immune modulatory effect.

Active suppression by Tregs plays a key role in the control of auto-reactive T cells and the induction of peripheral tolerance *in vivo.* In particular, the significance of Ag-specific Tregs in conferring resistance to organ-specific autoimmunity and in limiting autoimmune tissue damage has been documented in many disease models, including MS.⁴⁴ However, a safe and clinically feasible method for sustained expansion of endogenous Tregs has yet been identified.^{41, 44, 60, 63} a treatment protocol based on liver-directed AAV gene therapy can durably induce Ag-specific tolerance, thus having the potential of blocking the pathogenic autoimmune response present in MS and inhibiting disease activity; while avoiding the severe side effects associated with many of the currently used immunotherapies. Based on these and related studies, AAV8-liver gene transfer can restore immunological tolerance against myelin-sheath antigens, such as MOG and PLP, by inducing Ag-specific Tregs *in vivo.*

***Experimental approach and methods of analyses:*** This set of experiments tests vector constructs in order to verify efficiency of liver transduction and hepatic expression without adverse effects. Groups of (i) C57BL/6 or (ii) SJL/J mice (7-8 weeks old) will be injected with the 10¹¹ vector particles (vp) (effective dose of vector as previously determined) of (i) AAV8-MOG or (ii) AAV8-PLP (respectively), or control (irrelevant transgene, GFP) intravenously *via* the tail vein. Beginning on day 0, blood will be collected every 2 weeks and analyzed for the frequency of various T cell populations using standard markers of T cells phenotype (including, but not limited to, CD4, CD8, FoxP3, CD25, CD62L, CD44). Humoral immune responses (e.g. α-IgG1, -IgG2a, -IgG2c responses) may be determined *via* antigen specific ELISA. At 14 days post gene transfer, half of the mice from each group may be randomly selected and humanely euthanized. Tissues (blood, liver, spleen, and CNS (brain/spinal cord)) may be harvested for analysis. Hepatic transgene expression levels may be determined at the mRNA level using real-time quantitative PCR. Absolute and relative hepatic protein levels of the transgene will also be determined via western blot using liver lysates. At 90 days post injection, the remainder of the mice may be processed similarly to establish sustained transgene expression. Additionally, some mice may be subjected to EAE induction at various time points after vector administration and evaluated for prevention of disease, as described in preliminary data. Aliquots of the collected tissue samples may be archived as a reference material.

***In vitro functional suppression analysis of Ag-specific Tregs induced by AAV8 hepatic gene transfer.*** Splenic Tregs (CD4⁺CD25⁺) may be magnetically sorted from mice that received (i) AAV8-MOG or (ii) AAV8-PLP or AAV8-GFP (control) vector and co-cultured with graded numbers of CFSE labeled cells obtained from 2D2-TCR mice (this C57B1/6 mouse line expresses a TCR which recognize MOG₃₅₋₅₅ in the context of H-2 IA^{b}) or splenocytes harvested and labeled from SJL mice that have been previously immunized with PLP/adjuvant in the presence of anti-CD3/CD28 coated beads (provides APC independent/non-specific activation of Teff). Treg mediated suppression of proliferating effector cells may be determined by flow cytometry. Cell-culture supernatants may be analyzed for Th1/Th2/Th17 cytokines via specific assays. Results may be compared with data from naive and EAE induced mice (in which many CD4⁺CD25⁺ cells should represent activated effector rather than Treg). In combination, these studies will demonstrate Ag-specific functional suppression from the vector induced Tregs compared to controls.

Based on the initial data and published studies, the inventors expect maximal transgene expression by 2 weeks, that remains fairly unchanged over time, thus indicating stable transduction of hepatocytes.¹⁰ Since the vector constructs have been purposely designed to express full length MOG or PLP and include a strong hepatocyte promoter, it is also expected that AAV8 vector-mediated expression may be constrained to the hepatocytes and not secreted. Sequestering the transgene will constrain pathological consequences of freely circulating AAV-derived neuroantigen. Lastly, the inventors do not expect inflammatory responses in any tissues and analysis of liver enzymes (ALT/AST) should demonstrate an absence of hepatotoxicity. Furthermore, based on pilot studies, it is expected that vector administration prior to EAE induction will prevent disease development. A positive outcome would also be the absence/significant reduction in antigen specific antibody responses. Lastly, results from the Treg suppression assays are expected to show that suppression induced by hepatic transgene expression is facilitated by activation of Ag-specific Tregs.

Even though the literature overwhelmingly supports the idea that Tregs are potent suppressors of EAE and are the driving force to switch from disease progression to remission, very few studies in the past have addressed how to generate such Ag-specific Tregs that is both safe and effective.⁶⁴ In theory, this could be achieved by two approaches. The first would be to isolate Tregs, expand their numbers *ex vivo* then reintroduce them, with the idea that an increase in overall frequency of polyclonal Tregs might influence ongoing disease. In 2004, Bluestone's group in a type-1 diabetes model provided initial proof of principle for this approach.⁶⁵ More recently, others have further shown that using expanded Tregs from myelin-specific transgenic TCR mice is more effective.⁶⁴ The second approach is to administer a suitable treatment that promotes the expansion of Treg numbers and/or function *in vivo.* Recent reports have described the use of various compounds (e.g. nanoparticles/small molecules) to enhance Treg function in EAE, while others try to augment antigen presentation in order to generate Tregs.^{64, 66} In the end, a reliable and translatable method for induction of the disease relevant Ag-specific Tregs is still lacking-until now. This proposal presents a methodology that will provide a durable method for the continued *in vivo* induction of endogenous Ag-specific Tregs. Based on previous work, hepatic gene transfer using AAV8 vectors expressing full-length MOG or PLP should induce Ag-specific Tregs across multiple endogenous myelin epitopes in a manner that has been shown to be safe, feasible, and long-lasting.

***Experimental approach and methods of analyses.*** Here mice will first under go active induction of EAE using either (i) MOG or (ii) PLP. At the first clinical signs of EAE, in MOG induced chronic-progressive mice, or at the peak of disease, in PLP induced relapsing-remitting mice, AAV8-MOG or AAV8-PLP vector (respectively) or AAV8-GFP for control mice may be given. Mice may be clinically scored by weight and neurological deficit 2x daily. Blood may be collected and analyzed for humoral (IgG) responses as before. At ∼45 days, each cohort mice may be perfused and randomly subdivided into 2 groups. Group 1 will have brain, spinal cord, and liver tissues harvested and preserved for histopathological and immunofluorescent analysis. Infiltrating lymphocytes may be isolated from the brain and spinal cords from mice in Group 2 (as previously described⁶⁷). The frequency of various T cell populations may be analyzed using standard markers of T cells (including, but not limited to, CD4, CD8, FoxP3, CD25, CD62L, CD44, CTLA-4, CD103). Liver tissue may be subjected to transcriptional and protein analysis as shown. Results may be compared to control mice and reference material. Portions of the tissue may also be archived for future studies.

It is expected that therapeutic treatment with a single injection of AAV8-MOG or - PLP vector at the onset or peak of the disease will result in a dramatic remission in clinical impairment. There should be a concurrent reduction in antibody titers and/or frequency of B cell responses to the EAE inducing peptide, as compared to control-vector treated mice. CNS inflammation is characteristic of EAE, and the degree of lymphocyte infiltration correlates with disease progression; whereas, the presence of Tregs in the CNS during EAE has been associated with diminished inflammation and resolution of clinical disease.⁶⁸ Hence, it is expected that a significant reduction of inflammatory infiltrates in the CNS of vector treated mice upon histopathological analysis. This would suggest that the induced Ag-specific Tregs migrating to the site of CNS damage are protective and are capable limiting damage mediated by effector T cells. Additionally, the natural relapsing-remitting nature of PLP induced EAE may be exploited by timing the injection of the AAV8-PLP vector so that the peak effects of induced tolerance correspond to when the disease is relatively quiescent (remitting).

On the other hand, mice that receive MOG for EAE induction begin showing neurological impairments after ∼12 days that progressively escalate. In this scenario, it is possible that some level of inflammation will still be present, although; the phenotypic analysis of the T cells populations show that absolute numbers of T cells infiltrating the CNS to be lower with a greater Treg:Teff ratio.

***Transient immunosuppression using rapamycin.*** Rapamycin readily crosses the BBB thus exerting direct effects within the CNS. Blocking the activation of the mTOR pathway, rapamycin prevents activation of T cells by inhibiting their response to IL-2 thus preventing Ag-induced proliferation of Teff, while selectively allowing expansion of functional CD4⁺CD25⁺FoxP3⁺ Tregs. In EAE, rapamycin is effective in preventing the onset of disease, however; suppression of established disease is only maintained with continued use.⁶⁹ In a further series of experiments, vector treated mice are transiently immunosuppressed. Groups of mice are then injected with AAV8-MOG, -PLP, -GFP or PBS at specific time-points that correspond to either initial onset or peak of disease. Concurrently mice receive intraperitoneal rapamycin (1 mg/kg), or PBS (sham control) daily for 14 consecutive days.⁶⁹ At specific time points corresponding to pre- and post-treatment and significant changes in clinical scoring, tissues and lymphocytes may be harvested from the CNS and spleen from randomly selected mice. Histopathological changes within the tissues can then be identified. Isolated cells are then phenotyped and the frequency of Tregs and Teff from the different compartments may be determined and compared to control groups to validate the efficacy of rapamycin co-treatment.

Regardless of AAV8 administration, treatment with rapamycin alone is expected to transiently produce a rapid reduction in the clinical presentation of EAE because it selectively inhibits Teff proliferation.⁶⁹ However, when used in conjunction with AAV8 liver gene transfer, rapamycin treatment has a synergistic effect that results in an increase in vector induced Ag-specific FoxP3⁺ Tregs (since they are less sensitive to mTOR signaling inhibition) with a corresponding decrease in effector T cells.⁷⁰ The shift to tolerance is further potentiated by the fact Tregs have been shown to mediate selective inhibition of antigen-specific Th1 cells in the CNS of EAE.⁷¹

When considering the data clearly supports the ability of AAV liver gene transfer to induce Ag-specific Tregs and invoke immune tolerance; and that accumulation of Tregs in the CNS during the recovery phase of EAE has been a consistent finding in actively induced models, it seems unlikely that the present therapy would not have, at least to some degree, a clinical or pathological benefit. ^{3, 64, 71, 72}

In conclusion, the therapeutic regimen presented here addresses an unmet need by providing an effective treatment for diseases such as MS using a gene therapy approach. Using the AAV vector platform disclosed herein to deliver full-length proteins offers a superior HLA independent approach for Ag-specific Treg induction compared to other *ex vivo* or epitope restricted Treg mediated therapies. Additionally, AAV gene transfer results in continuous Treg generation because of the long-term hepatocyte expression of transgene.⁷³

### REFERENCES

The following references, to the extent that they provide exemplary procedural or other details supplementary to those set forth herein, are specifically incorporated herein by reference:
1. Kohm AP, Carpentier PA, Anger HA, Miller SD. Cutting edge: CD4+CD25+ regulatory T cells suppress antigen-specific autoreactive immune responses and central nervous system inflammation during active experimental autoimmune encephalomyelitis. Journal of Immunology (Baltimore, MD). 169(9):4712-4716 (2002).
2. Zhang G-X, Yu S, Gran B, Li J, Calida D, Ventura E, Chen X, Rostami A. T cell and antibody responses in remitting-relapsing experimental autoimmune encephalomyelitis in (C57BL/6×SJL) F1 mice. Journal of Neuroimmunology. 2004;148(1-2):1-10.
3. McGeachy MJ, Stephens LA, Anderton SM. Natural recovery and protection from autoimmune encephalomyelitis: contribution of CD4+CD25+ regulatory cells within the central nervous system. Journal of Immunology (Baltimore, MD), 2005;175(5):3025-3032.
4. Paust S, Cantor H. Regulatory T cells and autoimmune disease. Immunological reviews. 2005;204:195-207.
5. Marusic S, Tonegawa S. Tolerance induction and autoimmune encephalomyelitis amelioration after administration of myelin basic protein-derived peptide. Journal of Experimental Medicine. 1997;186(4):507-515.
6. LoDuca PA, Hoffman BE, Herzog RW. Hepatic gene transfer as a means of tolerance induction to transgene products. Current gene therapy. 2009;9(2):104-14.
7. Hoffman B, Dobrzynski E, Wang L, Hirao L, Mingozzi F, Cao O, Herzog RW. Muscle as a Target for Supplementary Factor IX Gene Transfer2007;18(7):603-13.
8. Dobrzynski E, Fitzgerald JC, Cao O, Mingozzi F, Wang L, Herzog RW. Prevention of cytotoxic T lymphocyte responses to factor IX-expressing hepatocytes by gene transfer-induced regulatory T cells. Proceedings of the National Academy of Sciences of the United States of America. 2006;103(12):4592-7.
9. Dobrzynski E, Herzog RW. Tolerance induction by viral in vivo gene transfer. Clinical medicine & research. 2005;3(4):234-40.
10. Cooper M, Nayak S, Hoffman BE, Terhorst C, Cao O, Herzog RW. Improved induction of immune tolerance to factor IX by hepatic AAV-8 gene transfer. Human gene therapy. 2009;20(7):767-76.
11. Cao O, Furlan-Freguia C, Arruda VR, Herzog RW. Emerging role of regulatory T cells in gene transfer. Curr Gene Ther. 2007;7(5):381-90.
12. Hoffman BE, Martino AT, Sack BK, Cao O, Liao G, Terhorst C, Herzog RW. Nonredundant roles of IL-10 and TGF-beta in suppression of immune responses to hepatic AAV-factor IX gene transfer. Molecular therapy : the journal of the American Society of Gene Therapy. 2011;19(7):1263-72.
13. Mingozzi F, Liu YL, Dobrzynski E, Kaufhold A, Liu JH, Wang Y, Arruda VR, High KA, Herzog RW. Induction of immune tolerance to coagulation factor IX antigen by in vivo hepatic gene transfer. The Journal of clinical investigation. 2003;111(9):1347-56. doi: 10.1172/JCI16887. PMID: 12727926.
14. Faust SM, Bell P, Zhu Y, Sanmiguel J, Wilson JM. The role of apoptosis in immune hyporesponsiveness following AAV8 liver gene transfer. Molecular therapy : the journal of the American Society of Gene Therapy. 2013;21(12):2227-35.
15. Markusic DM, Hoffman BE, Perrin GQ, Nayak S, Wang X, LoDuca PA, High KA, Herzog RW. Effective gene therapy for haemophilic mice with pathogenic factor IX antibodies. EMBO Molecular Medicine. 2013:1-12.
16. Annoni A, Brown BD, Cantore A, Sergi LS, Naldini L, Roncarolo MG. In vivo delivery of a microRNA-regulated transgene induces antigen-specific regulatory T cells and promotes immunologic tolerance. Blood. 2009;114(25):5152-61.
17. Breous E, Somanathan S, Vandenberghe LH, Wilson JM. Hepatic regulatory T cells and Kupffer cells are crucial mediators of systemic T cell tolerance to antigens targeting murine liver. Hepatology. 2009;50(2):612-21.
18. Matrai J, Cantore A, Bartholomae CC, Annoni A, Wang W, Acosta-Sanchez A, Samara-Kuko E, De Waele L, Ma L, Genovese P, Damo M, Arens A, Goudy K, Nichols TC, von Kalle C, MK LC, Roncarolo MG, Schmidt M, Vandendriessche T, Naldini L. Hepatocyte-targeted expression by integrase-defective lentiviral vectors induces antigen-specific tolerance in mice with low genotoxic risk. Hepatology. 2011;53(5):1696-707.
19. Mingozzi F, Hasbrouck NC, Basner-Tschakarjan E, Edmonson SA, Hui DJ, Sabatino DE, Zhou S, Wright JF, Jiang H, Pierce GF, Arruda VR, High KA. Modulation of tolerance to the transgene product in a nonhuman primate model of AAV-mediated gene transfer to liver. Blood. 2007;110(7):2334-41.
20. Sun B, Kulis MD, Young SP, Hobeika AC, Li S, Bird A, Zhang H, Li Y, Clay TM, Burks W, Kishnani PS, Koeberl DD. Immunomodulatory gene therapy prevents antibody formation and lethal hypersensitivity reactions in murine pompe disease. Molecular therapy : the journal of the American Society of Gene Therapy. 2010;18(2):353-360.
21. Miao CH, Harmeling BR, Ziegler SF, Yen BC, Torgerson T, Chen L, Yau RJ, Peng B, Thompson AR, Ochs HD, Rawlings DJ. CD4+FOXP3+ regulatory T cells confer long-term regulation of factor VIII-specific immune responses in plasmid-mediated gene therapy-treated hemophilia mice. Blood. 2009;114(19):4034-44.
22. Brown BD, Cantore A, Annoni A, Sergi LS, Lombardo A, Della Valle P, D'Angelo A, Naldini L. A microRNA-regulated lentiviral vector mediates stable correction of hemophilia B mice. Blood. 2007;110(13):4144-52.
23. Brown BD, Venneri MA, Zingale A, Sergi Sergi L, Naldini L. Endogenous microRNA regulation suppresses transgene expression in hematopoietic lineages and enables stable gene transfer. Nature medicine. 2006;12(5):585-91.
24. Cerullo V, McCormack W, Seiler M, Mane V, Cela R, Clarke C, Rodgers JR, Lee B. Antigen-specific tolerance of human alpha1-antitrypsin induced by helper-dependent adenovirus. Human gene therapy. 2007;18(12):1215-24.
25. Follenzi A, Battaglia M, Lombardo A, Annoni A, Roncarolo MG, Naldini L. Targeting lentiviral vector expression to hepatocytes limits transgene-specific immune response and establishes long-term expression of human antihemophilic factor IX in mice. Blood. 2004;103(10):3700-9.
26. Franco LM, Sun B, Yang X, Bird A, Zhang H, Schneider A, Brown T, Young SP, Clay TM, Amalfitano A, Chen YT, Koeberl DD. Evasion of immune responses to introduced human acid alpha-glucosidase by liver-restricted expression in glycogen storage disease type II. Molecular therapy: the journal of the American Society of Gene Therapy. 2005;12(5):876-84.
27. Koeberl DD, Kishnani PS. Immunomodulatory gene therapy in lysosomal storage disorders. Curr Gene Ther. 2009;9(6):503-10.
28. Luth S, Huber S, Schramm C, Buch T, Zander S, Stadelmann C, Bruck W, Wraith DC, Herkel J, Lohse AW. Ectopic expression of neural autoantigen in mouse liver suppresses experimental autoimmune neuroinflammation by inducing antigen-specific Tregs. The Journal of clinical investigation. 2008;118(10):3403-10.
29. Matsui H, Hegadorn C, Ozelo M, Burnett E, Tuttle A, Labelle A, McCray PB, Jr., Naldini L, Brown B, Hough C, Lillicrap D. A microRNA-regulated and GP64-pseudotyped lentiviral vector mediates stable expression of FVIII in a murine model of Hemophilia A. Molecular therapy: the journal of the American Society of Gene Therapy. 2011;19(4):723-30.
30. Matsui H, Shibata M, Brown B, Labelle A, Hegadorn C, Andrews C, Chuah M, VandenDriessche T, Miao CH, Hough C, Lillicrap D. A murine model for induction of long-term immunologic tolerance to factor VIII does not require persistent detectable levels of plasma factor VIII and involves contributions from Foxp3+ T regulatory cells. Blood. 2009;114(3):677-85.
31. McEachern KA, Nietupski JB, Chuang WL, Armentano D, Johnson J, Hutto E, Grabowski GA, Cheng SH, Marshall J. AAV8-mediated expression of glucocerebrosidase ameliorates the storage pathology in the visceral organs of a mouse model of Gaucher disease. Journal of Gene Medicine, 2006;8(6):719-29.
32. Nietupski JB, Hurlbut GD, Ziegler RJ, Chu Q, Hodges BL, Ashe KM, Bree M, Cheng SH, Gregory RJ, Marshall J, Scheule RK. Systemic administration of AAV8-alpha-galactosidase A induces humoral tolerance in nonhuman primates despite low hepatic expression. Molecular therapy : the journal of the American Society of Gene Therapy. 2011;19(11):1999-2011.
33. Passini MA, Bu J, Fidler JA, Ziegler RJ, Foley JW, Dodge JC, Yang WW, Clarke J, Taksir TV, Griffiths DA, Zhao MA, O'Riordan CR, Schuchman EH, Shihabuddin LS, Cheng SH. Combination brain and systemic injections of AAV provide maximal functional and survival benefits in the Niemann-Pick mouse. Proceedings of the National Academy of Sciences of the United States of America. 2007;104(22):9505-10.
34. Sharland A, Logan GJ, Bishop A, Alexander IE. Liver-directed gene expression using recombinant AAV 2/8 vectors--a tolerogenic strategy for gene delivery? Discovery medicine. 2010;9(49):519-27.
35. Somanathan S, Breous E, Bell P, Wilson JM. AAV vectors avoid inflammatory signals necessary to render transduced hepatocyte targets for destructive T cells. Molecular therapy : the journal of the American Society of Gene Therapy. 2010;18(5):977-82.
36. Sun B, Bird A, Young SP, Kishnani PS, Chen YT, Koeberl DD. Enhanced response to enzyme replacement therapy in Pompe disease after the induction of immune tolerance. American journal of human genetics. 2007;81(5):1042-9.
37. Ziegler RJ, Lonning SM, Armentano D, Li C, Souza DW, Cherry M, Ford C, Barbon CM, Desnick RJ, Gao G, Wilson JM, Peluso R, Godwin S, Carter BJ, Gregory RJ, Wadsworth SC, Cheng SH. AAV2 vector harboring a liver-restricted promoter facilitates sustained expression of therapeutic levels of alpha-galactosidase A and the induction of immune tolerance in Fabry mice. Molecular therapy : the journal of the American Society of Gene Therapy. 2004;9(2):231-40.
38. Moses H, Picone MA, Smith V. An In-Depth Overview: Consensus Medical Communications; 2012.
39. Gonsette RE. Self-tolerance in multiple sclerosis. Acta neurologica Belgica. 2012;112(2):133-40
40. Viglietta V, Baecher-Allan C, Weiner HL, Hafler DA. Loss of functional suppression by CD4+CD25+ regulatory T cells in patients with multiple sclerosis. J Exp Med. 2004;199(7):971-9
41. Zozulya AL, Wiendl H. The role of regulatory T cells in multiple sclerosis. Nature Clinical Practice Neurology. 2008;4(7):384-98.
42. Buckner JH. Mechanisms of impaired regulation by CD4(+)CD25(+)FOXP3(+) regulatory T cells in human autoimmune diseases. Nature Reviews Immunology. 2010;10(12):849-59. doi: 10.1038/nri2889.
43. Buc M. Role of Regulatory T Cells in Pathogenesis and Biological Therapy of Multiple Sclerosis. Mediators of Inflammation. 2013;2013(6): 1-11.
44. Costantino CM, Baecher-Allan C, Hafler DA. Multiple Sclerosis and Regulatory T Cells. Journal of Clinical Immunology. 2008;28(6):697-706.
45. Hoffman BE, Herzog RW. Coaxing the liver into preventing autoimmune disease in the brain. The Journal of clinical investigation. 2008;118(10):3271-3.
46. Lüth S, Huber S, Schramm C, Buch T, Zander S, Stadelmann C, Brück W, Wraith DC, Herkel J, Lohse AW. Ectopic expression of neural autoantigen in mouse liver suppresses experimental autoimmune neuroinflammation by inducing antigen-specific Tregs. The Journal of clinical investigation. 2008;118(10):3403-10.
47. Haas J, Korporal M, Balint B, Fritzsching B, Schwarz A, Wildemann B. Glatiramer acetate improves regulatory T-cell function by expansion of naive CD4(+)CD25(+)FOXP3(+)CD31(+) T-cells in patients with multiple sclerosis. J Neuroimmunol. 2009;216(1-2):113-7.
48. Nathwani AC, Tuddenham EG, Rangarajan S, Rosales C, McIntosh J, Linch DC, Chowdary P, Riddell A, Pie AJ, Harrington C, O'Beirne J, Smith K, Pasi J, Glader B, Rustagi P, Ng CY, Kay MA, Zhou J, Spence Y, Morton CL, Allay J, Coleman J, Sleep S, Cunningham JM, Srivastava D, Basner-Tschakarjan E, Mingozzi F, High KA, Gray JT, Reiss UM, Nienhuis AW, Davidoff AM. Adenovirus-associated virus vector-mediated gene transfer in hemophilia B. The New England journal of medicine. 2011;365(25):2357-65.
49. Herzog RW, Cao O, Srivastava A. Two decades of clinical gene therapy--success is finally mounting. Discovery medicine. 2010;9(45):105-11. Epub 2010/03/03.
50. Mingozzi F, High KA. Therapeutic in vivo gene transfer for genetic disease using AAV: progress and challenges. Nat Rev Genet. 2011;12(5):341-55.
51. Maclaren RE, Groppe M, Barnard AR, Cottriall CL, Tolmachova T, Seymour L, Clark KR, During MJ, Cremers FP, Black GC, Lotery AJ, Downes SM, Webster AR, Seabra MC. Retinal gene therapy in patients with choroideremia: initial findings from a phase 1/2 clinical trial. Lancet. 2014. Epub 2014/01/21.
52. Bryant LM, Christopher DM, Giles AR, Hinderer C, Rodriguez JL, Smith JB, Traxler EA, Tycko J, Wojno AP, Wilson JM. Lessons learned from the clinical development and market authorization of Glybera. Human gene therapy Clinical development. 2013;24(2):55-64.
53. Byrne BJ, Falk DJ, Pacak CA, Nayak S, Herzog RW, Elder ME, Collins SW, Conlon TJ, Clement N, Cleaver BD, Cloutier DA, Porvasnik SL, Islam S, Elmallah MK, Martin A, Smith BK, Fuller DD, Lawson LA, Mah CS. Pompe disease gene therapy. Human molecular genetics. 2011;20(R1):R61-R68.
54. Moghimi B, Sack BK, Nayak S, Markusic DM, Mah CS, Herzog RW. Induction of tolerance to factor VIII by transient co-administration with rapamycin. Journal of thrombosis and haemostasis: JTH. 2011;9(8):1524-1533.
55. Nayak S, Cao O, Hoffman BE, Cooper M, Zhou S, Atkinson MA, Herzog RW. Prophylactic immune tolerance induced by changing the ratio of antigen-specific effector to regulatory T cells. Journal of thrombosis and haemostasis : JTH. 2009;7(9):1523-1532.
56. Nayak S, Sarkar D, Perrin GQ, Moghimi B, Hoffman BE, Zhou S, Byrne BJ, Herzog RW. Prevention and Reversal of Antibody Responses Against Factor IX in Gene Therapy for Hemophilia B. Frontiers in Microbiology. 2011;2.
57. Martino AT, Nayak S, Hoffman BE, Cooper M, Liao G, Markusic DM, Byrne BJ, Terhorst C, Herzog RW. Tolerance induction to cytoplasmic beta-galactosidase by hepatic AAV gene transfer: implications for antigen presentation and immunotoxicity. PloS one. 2009;4(8):e6376.
58. Lalive PH, Molnarfi N, Benkhoucha M, Weber MS, Santiago-Raber ML. Antibody response in MOG(35-55) induced EAE. J Neuroimmunol. 2011;240-241:28-33. doi: 10.1016/j.jneuroim.2011.09.005.
59. Mann MK, Ray A, Basu S, Karp CL, Dittel BN. Pathogenic and regulatory roles for B cells in experimental autoimmune encephalomyelitis. Autoimmunity. 2012;45(5):388-399.
60. Miller SD, Karpus WJ, Davidson TS. Experimental autoimmune encephalomyelitis in the mouse. Current protocols in immunology / edited by John E Coligan [et al]. 2010;Chapter 15:Unit 15.1.
61. Ko HJ, Chung JY, Nasa Z, Chan J, Siatskas C, Toh BH, Alderuccio F. Targeting MOG expression to dendritic cells delays onset of experimental autoimmune disease. Autoimmunity. 2011;44(3):177-87.
62. Dobrzynski E, Mingozzi F, Liu YL, Bendo E, Cao O, Wang L, Herzog RW. Induction of antigen-specific CD4+ T-cell anergy and deletion by in vivo viral gene transfer. Blood. 2004;104(4):969-77.
63. Fissolo N, Costa C, Nurtdinov RN, Bustamante MF, Llombart V, Mansilla MJ, Espejo C, Montalban X, Comabella M. Treatment with MOG-DNA vaccines induces CD4+CD25+FoxP3+ regulatory T cells and up-regulates genes with neuroprotective functions in experimental autoimmune encephalomyelitis. Journal of neuroinflammation. 2012;9:139.
64. O'Connor RA, Anderton SM. Foxp3+ regulatory T cells in the control of experimental CNS autoimmune disease. J Neuroimmunol. 2008;193(1-2):1-11.
65. Tang Q, Henriksen KJ, Bi M, Finger EB, Szot G, Ye J, Masteller EL, McDevitt H, Bonyhadi M, Bluestone JA. In vitro-expanded antigen-specific regulatory T cells suppress autoimmune diabetes. J Exp Med. 2004;199(11):1455-65.
66. Chen X, Oppenheim JJ, Winkler-Pickett RT, Ortaldo JR, Howard OM. Glucocorticoid amplifies IL-2-dependent expansion of functional FoxP3(+)CD4(+)CD25(+) T regulatory cells in vivo and enhances their capacity to suppress EAE. European journal of immunology. 2006;36(8):2139-49.
67. Oleszak EL, *Hoffman BE, Chang JR, Zaczynska E, Gaughan J, Katsetos CD, Platsoucas CD, Harvey N. Apoptosis of infiltrating T cells in the central nervous system of mice infected with Theiler's murine encephalomyelitis virus. Virology. 2003;315(1):110-23. Epub 2003/11/01.
68. Selvaraj RK, Geiger TL. Mitigation of experimental allergic encephalomyelitis by TGF-beta induced Foxp3+ regulatory T lymphocytes through the induction of anergy and infectious tolerance. Journal of immunology. 2008;180(5):2830-8.
69. Esposito M, Ruffini F, Bellone M, Gagliani N, Battaglia M, Martino G, Furlan R. Rapamycin inhibits relapsing experimental autoimmune encephalomyelitis by both effector and regulatory T cells modulation. J. Neuroimmunol., 2010;220(1-2):52-63.
70. Bensinger SJ, Walsh PT, Zhang J, Carroll M, Parsons R, Rathmell JC, Thompson CB, Burchill MA, Farrar MA, Turka LA. Distinct IL-2 receptor signaling pattern in CD4+CD25+ regulatory T cells. Journal of immunology. 2004; 172(9):5287-96.
71. O'Connor RA, Malpass KH, Anderton SM. The inflamed central nervous system drives the activation and rapid proliferation of Foxp3+ regulatory T cells. Journal of immunology. 2007;179(2):958-66.
72. Korn T, Reddy J, Gao W, Bettelli E, Awasthi A, Petersen TR, Backstrom BT, Sobel RA, Wucherpfennig KW, Strom TB, Oukka M, Kuchroo VK. Myelin-specific regulatory T cells accumulate in the CNS but fail to control autoimmune inflammation. Nature medicine. 2007;13(4):423-31.
73. Niemeyer GP, Herzog RW, Mount J, Arruda VR, Tillson DM, Hathcock J, van Ginkel FW, High KA, Lothrop CD, Jr. Long-term correction of inhibitor-prone hemophilia B dogs treated with liver-directed AAV2-mediated factor IX gene therapy. Blood. 2009 113(4):797-806.

### EXAMPLE 4 - FURTHER DATA FROM EAE MOUSE MODEL AND ASSESSMENT OF OTHER PROTEINS

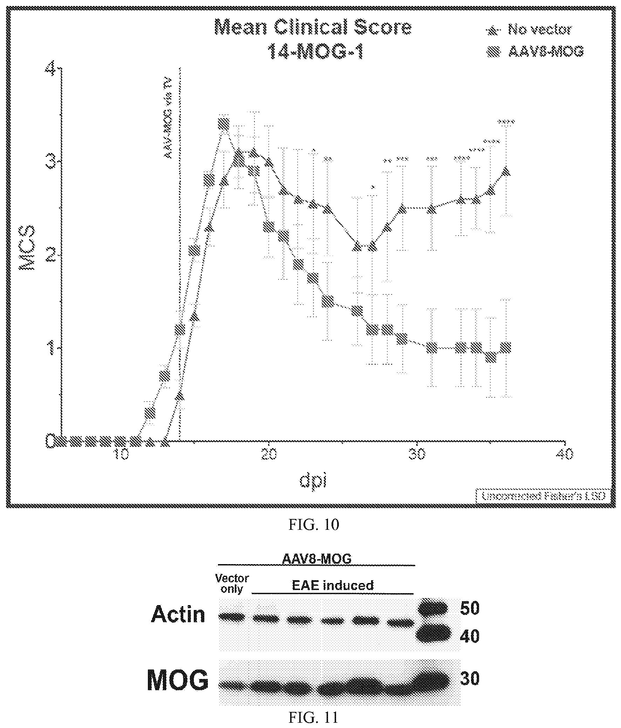

Animals were injected intravenously via tail vein with 10¹¹vector particles of AAV8-apoE/hAAT-MOG. The MOG sequence used was murine MOG. It was shown that MOG transgene was expressed in the liver as evidenced increased amounts of MOG protein (FIG. 11) in samples from the liver.

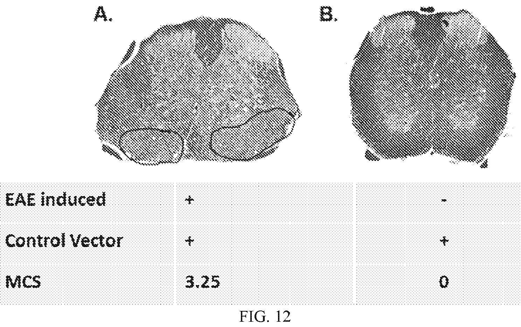

To ensure that AAV did not interfere with the development or progression of EAE in the mouse model described in the other Examples, control AAV8-GFP was injected intravenously into mice. Two weeks later, EAE was induced or not induced. The AAV control vector did not appear to interfere with development or progression of EAE (FIG. 12).

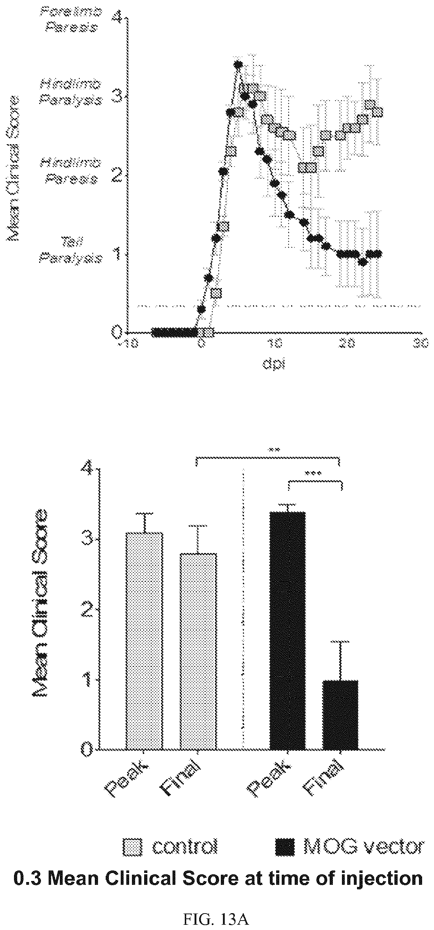

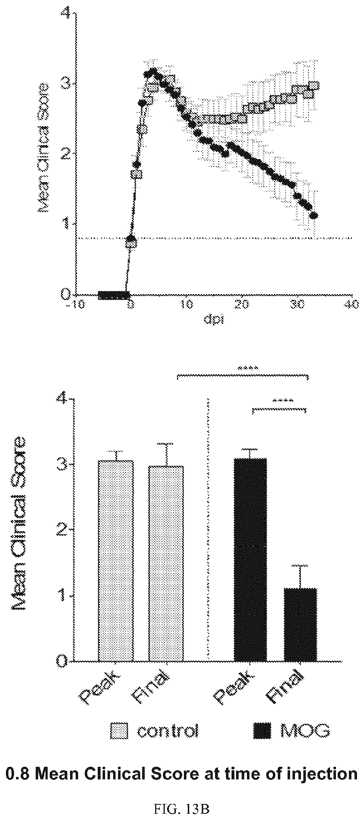

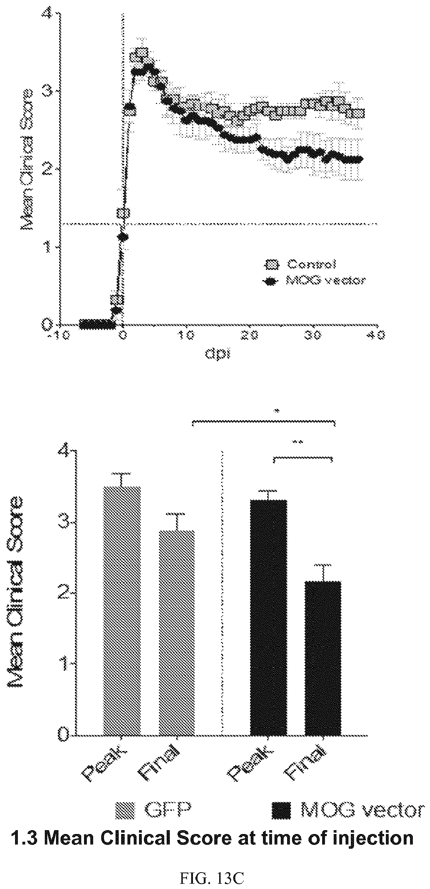

In another study, EAE was induced in C57BL/6 mice. At various times of neurological deficit of mean clinical score (MCS) ∼0.3, ∼0.8, or ∼1.3, mice received AAV8-MOG or control vector. Mean clinical score was recorded. Even at increasing disease pathology, AAV-MOG vector had significantly reduced neurological deficit compared to control vector treated mice (FIGs. 13A-C). Bar graphs show statistical significance between final scores and peak-to-final scores.

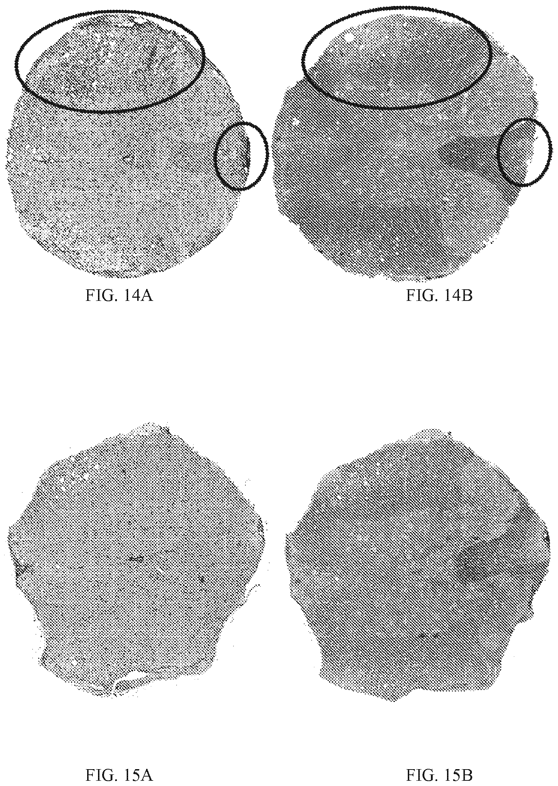

In a further study, serial sections of spinal cord were taken from an EAE induced female mouse ∼35 days after receiving control vector (MCS=4.0) . Hematoxylin and eosin stain showed areas of high inflammatory infiltration (FIG. 14A). Luxol fast blue stain showed areas of demyelination (FIG. 14B). In contrast, serial sections of spinal cord from an EAE induced female mouse ∼35 days after receiving AAV-MOG vector (MCS =1.25) showed suppression of inflammation. Hematoxylin and eosin stain showed diminished infiltration (FIG. 15A). Luxol fast blue stain appeared to have less areas of demyelination as a result of the suppression of the inflammation (FIG. 15B).

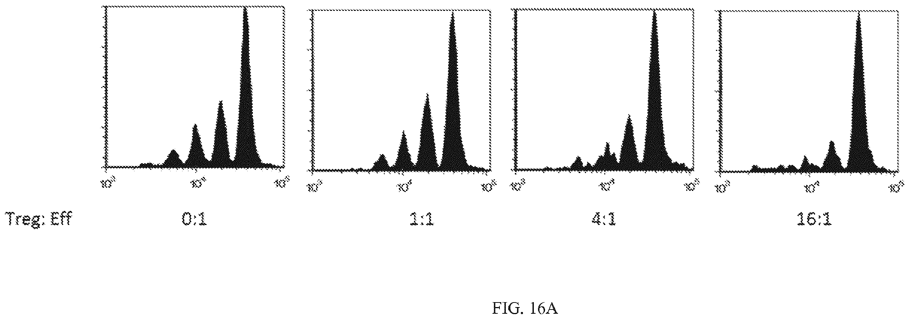

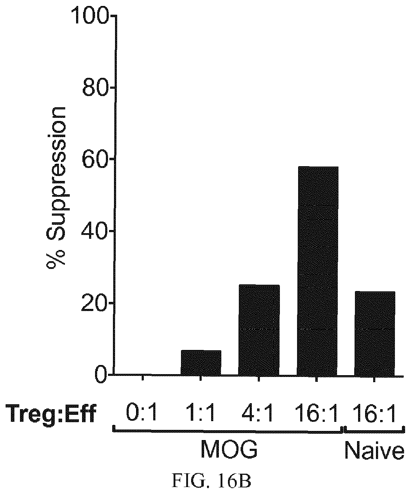

In another study, regulatory T cell (Treg)-mediated suppression was measured by CFDA-SE Cell Tracer . Effector T cells (Teff) were isolated from C57BL/6 mice and labeled with CFDA. Cells were cultured either alone or in the presence of Tregs at a various Treg:Teff ratios. After 72 hours, proliferation was determined by CFDA dilution and flow cytometric analysis. Tregs isolated from spleens of AAV-MOG treated mice were found to be functionally suppressive (FIG. 16A and 16B).

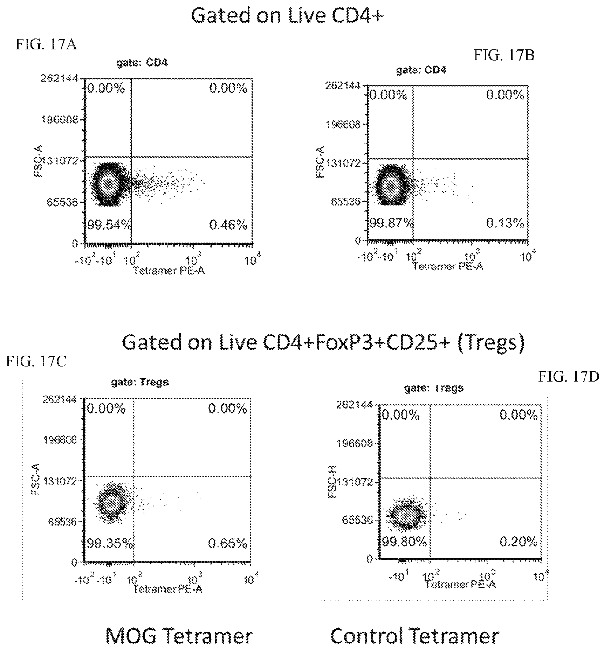

In a further study, the ability of AAV-MOG to induce antigen specific Tregs was assessed. Splenocytes from mice injected with AAV-MOG vector 8 weeks prior showed an increase in frequencies of I-Ab MOG₃₅₋₅₅ Tetramer positive CD4+ and Treg+ compared to control tetramer (FIGs. 17A-D), indicating that AAV-MOG vector induced antigen specific Tregs.

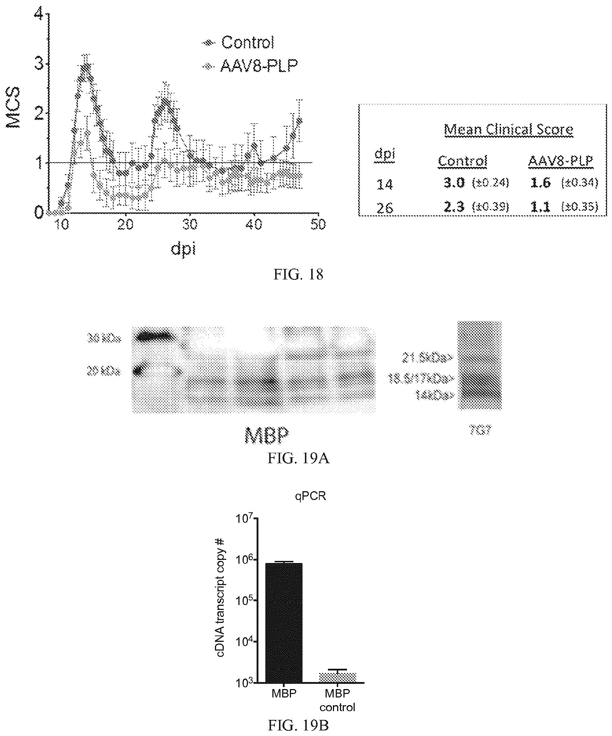

Next, a PLP vector was tested. AAV8-PLP was used for this part of the study. The PLP used was murine PLP. This initial proof-of-concept experiment demonstrated the timeline and clinical scoring for successful induction of PLP/EAE and the potential therapeutic benefit of liver gene transfer. Female SJL mice were injected with AAV8-PLP or control 2 weeks before immunization with 200µg PLP emulsified in CFA containing 4mg/ml Mycobacterium tuberculosis. Clinical signs of EAE began ∼10 days later at which time mice were evaluated twice daily. Mice were scored according to the severity of the clinical signs (FIG. 2). Clearly, mice receiving AAV8-PLP vector had a significant reduction in disease at the peak of onset (FIG. 18). There was also a significant decrease in MCS during relapse (day 26) with fewer relapses overall (FIG. 18). These results show that AAV8-PLP reduced clinical severity in mice with PLP induced relapsing-remitting EAE. Increased reduction or complete prevention is anticipated with optimization of vector dose and timing.

Lastly, a MBP vector was tested. AAV8-MBP was used for this part of the study. The MBP used was murine MBP. Western blot analysis from protein extracted from liver of mice injected with AAV8-MBP showed an increase in MBP expression (FIG. 19A), which was consistent with an increase in mRNA levels (FIG. 19B).

It should be understood that the examples and embodiments described herein are for illustrative purposes only and that various modifications or changes in light thereof may be suggested to persons skilled in the art and are to be included within the spirit and purview of this application and the scope of the appended claims. All references, including publications, patent applications and patents, cited herein are hereby incorporated by reference to the same extent as if each reference was individually and specifically indicated to be incorporated by reference and was set forth in its entirety herein. Recitation of ranges of values herein are merely intended to serve as a shorthand method of referring individually to each separate value falling within the range, unless otherwise indicated herein, and each separate value is incorporated into the specification as if it were individually recited herein.

The description herein of any aspect or embodiment of the invention using terms such as "comprising", "having", "including" or "containing" with reference to an element or elements is intended to provide support for a similar aspect or embodiment of the invention that "consists of', "consists essentially of', or "substantially comprises" that particular element or elements, unless otherwise stated or clearly contradicted by context (*e.g.,* a composition described herein as comprising a particular element should be understood as also describing a composition consisting of that element, unless otherwise stated or clearly contradicted by context).

All of the compositions and methods disclosed and claimed herein can be made and executed without undue experimentation in light of the present disclosure. While the compositions and methods of this invention have been described in terms of preferred embodiments, it will be apparent to those of skill in the art that variations may be applied to the compositions and methods and in the steps or in the sequence of steps of the method described herein without departing from the concept, spirit and scope of the invention. More specifically, it will be apparent that certain agents that are chemically and/or physiologically related may be substituted for the agents described herein while the same or similar results would be achieved. All such similar substitutes and modifications apparent to those skilled in the art are deemed to be within the spirit, scope and concept of the invention as defined by the appended claims.

Embodiments of the invention will now be described in the following numbered paragraphs:
1. A recombinant adeno-associated viral (rAAV) nucleic acid vector comprising:
   a polynucleotide that includes a nucleic acid segment that encodes a first autoimmune disease therapeutic molecule operably linked to a promoter that is capable of expressing the nucleic acid segment in one or more cells of a mammalian liver.
2. The rAAV nucleic acid vector of paragraph 1, wherein the nucleic acid segment encodes a mammalian myelin basis protein (MBP), proteolipid protein (PLP), or myelin oligodendrocyte glycoprotein (MOG).
3. The rAAV nucleic acid vector of paragraph 1, wherein the nucleic acid segment encodes a human myelin basis protein (MBP), proteolipid protein (PLP), or myelin oligodendrocyte glycoprotein (MOG).
4. The rAAV nucleic acid vector of paragraph 2 or 3, wherein the nucleic acid segment encodes a biologically-active myelin basis protein (MBP), proteolipid protein (PLP), or myelin oligodendrocyte glycoprotein (MOG), that comprises an at least 20 amino acid contiguous sequence as set forth in SEQ ID NOs: 1, 2, 3, 9, 11, 13, 15, 17, 19, 21, 23, 25, 27, 29, 31, 33, or 35.
5. The rAAV nucleic acid vector of paragraph 4, wherein the nucleic acid segment encodes a biologically-active myelin basis protein (MBP), proteolipid protein (PLP), or myelin oligodendrocyte glycoprotein (MOG), comprises an amino acid sequence that is at least 95% identical to the sequence of SEQ ID NOs: 1, 2, 3, 9, 11, 13, 15, 17, 19, 21, 23, 25, 27, 29, 31, 33, or 35.
6. The rAAV nucleic acid vector of paragraph 2, wherein the nucleic acid segment encodes a biologically-active myelin basis protein (MBP), proteolipid protein (PLP), or myelin oligodendrocyte glycoprotein (MOG), that comprises an at least 20 amino acid contiguous sequence as set forth in SEQ ID NO:1, SEQ ID NO:2, or SEQ ID NO:3, respectively.
7. The rAAV nucleic acid vector of paragraph 6, wherein the nucleic acid segment encodes a biologically-active myelin basis protein (MBP), proteolipid protein (PLP), or myelin oligodendrocyte glycoprotein (MOG) that comprises an amino acid sequence that is at least 95% identical to the sequence of SEQ ID NO:1, SEQ ID NO:2, or SEQ ID NO:3.
8. The rAAV nucleic acid vector of any one of paragraphs 1 to 7, wherein the nucleic acid segment further comprises an enhancer, a post-transcriptional regulatory sequence, a polyadenylation signal, or any combination thereof, operably linked to the nucleic acid segment encoding the therapeutic molecule.
9. The rAAV nucleic acid vector of any one of paragraphs 1 to 8, wherein the promoter is a mammalian cell-specific or a mammalian tissue-specific promoter.
10. The rAAV nucleic acid vector of any one of paragraphs 1 to 9, wherein the nucleic acid segment further encodes or further expresses a polypeptide, a peptide, a ribozyme, a peptide nucleic acid, an siRNA, an RNAi, an antisense oligonucleotide, an antisense polynucleotide, an antibody, an antigen binding fragment, or any combination thereof.
11. The rAAV nucleic acid vector of paragraph 10, wherein the nucleic acid segment further encodes a second distinct therapeutic molecule that is effective in treating or ameliorating one or more symptoms of autoimmune disease in the mammal.
12. The rAAV nucleic acid vector of paragraph 11, wherein the autoimmune disease is multiple sclerosis.
13. A rAAV particle comprising the rAAV nucleic acid vector of any one of paragraphs 1 to 12.
13. A method for providing a mammal in need thereof with a therapeutically-effective amount of a selected therapeutic agent, the method comprising systemically administering to the mammal, an amount of the rAAV nucleic acid vector of any one of paragraphs 1 to 12 or the rAAV particle of paragraph 13; and for a time effective to provide the mammal with a therapeutically-effective amount of the encoded therapeutic molecule.
14. A method for treating or ameliorating one or more symptoms of an inflammatory disease in a mammal, the method comprising: systemically administering to the mammal, the rAAV nucleic acid vector of any one of paragraphs 1 to 12 or the rAAV particle of paragraph 13, in an amount and for a time sufficient to treat or ameliorate the one or more symptoms of autoimmune disease in the mammal.
15. The method of paragraph 14, wherein the mammal has, is suspected of having, is at risk for developing, or has been diagnosed with at least a first autoimmune disorder.
16. The method of paragraph 15, wherein the autoimmune disease is multiple sclerosis, disseminated sclerosis, or encephalomyelitis disseminata.
17. The method of paragraph 16, wherein the mammal is a newborn, an infant, a juvenile, or a young adult.
18. The method of any one of paragraphs 14 to 17, wherein production of the therapeutic molecule in the mammal reduces CNS inflammation, inhibits demyelination, reestablishes immune tolerance to one or more neuroproteins, stimulates the production of endogenous antigen-specific regulatory T cells, or any combination thereof.
19. The method of any one of paragraphs 14 to 18, wherein the rAAV vector encodes a second distinct therapeutic molecule selected from the group consisting of an agonist, an antagonist, an anti-apoptosis factor, an inhibitor, a receptor, a cytokine, a cytotoxin, an erythropoietic agent, a glycoprotein, a growth factor, a growth factor receptor, a hormone, a hormone receptor, an interferon, an interleukin, an interleukin receptor, a nerve growth factor, a neuroactive peptide, a neuroactive peptide receptor, a protease, a protease inhibitor, a protein decarboxylase, a protein kinase, a protein kinsase inhibitor, an enzyme, a receptor binding protein, a transport protein or an inhibitor thereof, a serotonin receptor, or an uptake inhibitor thereof, a serpin, a serpin receptor, a tumor suppressor, a chemotherapeutic, or any combination thereof.

## Claims

1. A recombinant adeno-associated viral (rAAV) nucleic acid vector comprising: a polynucleotide that comprises a nucleic acid segment that encodes a first autoimmune disease therapeutic molecule, wherein the nucleic acid segment is operably linked to a hepatocyte specific promoter that expresses the autoimmune disease therapeutic molecule in liver,
wherein the rAAV nucleic acid vector is encapsidated by an rAAV particle of serotype AAV1, AAV3, AAV4, AAV5, AAV6, AAV7, AAV8, AAV9, AAV10, AAV2-AAV3 hybrid, AAVrh.10, AAVhu.14, AAV3a/3b, AAVrh32.33, AAV-HSC15, AAV-HSC17, AAVhu.37, AAVrh.8, CHt-P6, AAV2.5, AAV6.2, AAV2i8, AAV-HSC15/17, AAVM41, AAV9.45, AAV6(Y445F/Y731F), AAV2.5T, AAV-HAE1/2, AAV clone 32/83, AAVShHIO, AAV2(Y→F), AAV8(Y733F), AAV2.15, AAV2.4, AAVM41, or AAVr3.45; or a derivative thereof.

2. The rAAV nucleic acid vector of claim 1, wherein the rAAV particle is of serotype AAV8, or wherein the AAV particle is pseudotyped.

3. The rAAV nucleic acid vector of claim 1 or 2, wherein the nucleic acid segment further encodes a second autoimmune disease therapeutic molecule, optionally wherein the nucleic acid segment further encodes a third autoimmune disease therapeutic molecule.

4. The rAAV nucleic acid vector of any one of claims 1-3, wherein the polynucleotide comprises AAV2 inverted terminal repeat sequences (ITRs).

5. The rAAV nucleic acid vector of any one of claims 1-4, wherein the hepatocyte specific promoter comprises an albumin promoter, a human α₁-antitrypsin promoter, a transthyretin (TTR) promoter, or an apolipoprotein E (apoE) promoter.

6. The rAAV nucleic acid vector of any one of claims 1-5, wherein the first, second and/or third autoimmune disease therapeutic molecule comprises a myelin basic protein, a proteolipid protein, a myelin oligodendrocyte, a glycoprotein, a myelin-associated glycoprotein, insulin, an islet-specific glucose-6-phosphatase catalytic subunit-related protein, a Preproinsulin (e.g., for type 1 diabetes), a glutamic decarboxylase, a tyrosine phosphatase like autoantigen, an insulinoma antigen- 2, an Islet cell antigen, a thyroid stimulating hormone (TSH) receptor, a thyrotropin receptor, an Aggrecan, a CD4+T cell epitope, or an acetylcholine receptor, optionally wherein the first autoimmune disease therapeutic molecule comprises a myelin basic protein (MBP), a proteolipid protein (PLP), or a myelin oligodendrocyte glycoprotein (MOG).

7. The rAAV nucleic acid vector of any one of claims 1-6, wherein the first autoimmune disease therapeutic molecule comprises MBP, PLP or MOG, optionally wherein the second autoimmune disease therapeutic molecule comprises MBP, PLP or MOG.

8. The rAAV nucleic acid vector of any one of claims 1-7, wherein the nucleic acid segment encodes MBP, PLP, and MOG.

9. The rAAV nucleic acid vector of any one of claims 1-8, wherein the first, second and/or third autoimmune disease therapeutic molecule is a human protein.

10. The rAAV nucleic acid vector of any one of claims 1-9, wherein the first autoimmune disease therapeutic molecule is encoded by a nucleotide sequence that is at least 95% identical to any one of SEQ ID NOs: 8, 10, 12, 14, 16, 18, 20, 22, 24, 26, 28, 30, 32, and 34, optionally wherein the first autoimmune disease therapeutic molecule is encoded by a nucleotide sequence of any one of SEQ ID NOs: 8, 10, 12, 14, 16, 18, 20, 22, 24, 26, 28, 30, 32, and 34.

11. The rAAV nucleic acid vector of any one of claims 1-9, wherein the first autoimmune disease therapeutic molecule comprises an amino acid sequence that is at least 95% identical to any one of SEQ ID NOs: 1, 2, 3, 9, 11, 13, 15, 17, 19, 21, 23, 25, 27, 29, 31, 33, or 35, optionally wherein the first autoimmune disease therapeutic molecule comprises an amino acid sequence of any one of SEQ ID NOs: 1, 2, 3, 9, 11, 13, 15, 17, 19, 21, 23, 25, 27, 29, 31, 33, or 35.

12. A pharmaceutical composition comprising an effective amount of the rAAV nucleic acid vector of any one of claims 1-11, optionally wherein the composition further comprises a pharmaceutically acceptable carrier.

13. A kit comprising the pharmaceutical composition of claim 12 and an mTOR inhibitor, optionally wherein the mTOR inhibitor is rapamycin.

14. An rAAV nucleic acid vector according to any one of claims 1-11 or a pharmaceutical composition according to claim 12 for use in medicine.

15. An rAAV particle comprising the rAAV nucleic acid vector of any one of claims 1-11 or a pharmaceutical composition according to claim 12 for use in treating or ameliorating one or more symptoms of an autoimmune disease in a mammal, optionally wherein the autoimmune disease is multiple sclerosis.
